# EUROPEAN PATENT APPLICATION

(11) **EP 4 585 175 A2**
(43) Date of publication of application: **16.07.2025**
(21) Application number: 25179656.1
(22) Date of filing: 28.03.2023
(51) Int. Cl.: A61B 18/00

(54) **SURGICAL SYSTEM FOR EXCISING A VALVE**

(30) Priority: 28.03.2022 US 202263324413 P; 15.11.2022 US 202263425507 P
(62) Divisional of application: 23719536.7
(71) Applicant: Excision Medical, Inc., Malvern, PA 19355 (US)
(72) Inventor: WALTERS, Greg, Alan, Malvern, 19355 (US); JOUIN, Francois, Malvern, 19355 (US); DOTSEY, Michael, A., Malvern, 19355 (US); CHERIAN, Shawn, Sabu, Malvern, 19355 (US); KANE, Alison, Mikayla, Malvern, 19355 (US); SATHANANTHAN, Janarthanan, Malvern, 19355 (US); SMITH, Chad, J., Malvern, 19355 (US)
(74) Representative: Haseltine Lake Kempner LLP

(57) **Abstract**

The present disclosure describes a surgical system for an aortic or mitral valve in a heart. The surgical system is configured to pierce, seize, and/or cut a leaflet, and/or facilitate removal of an excised portion of a leaflet.

## Description

### CROSS-REFERENCE TO RELATED APPLICATIONS

The present application claims priority to and the benefit of U.S. Provisional Application No. 63/324,413, filed March 28, 2022, and U.S. Provisional Application No. 63/425,507, filed November 15, 2022, the entire contents of which incorporated by reference into the present application for all purposes.

### TECHNICAL FIELD

The present disclosure relates to a surgical system for excising a portion of a native heart valve or an implanted heart valve.

### BACKGROUND

Transcatheter aortic valve replacement (TAVR) is an alternative option for the treatment of patients with severe calcific aortic stenosis. Indeed, TAVR may become the preferred therapy for all patients irrespective of surgical risk. However, transcatheter heart valves (THV) may fail in the future and repeat intervention may be required. So-called redo-transcatheter aortic valve implantation (TAVI) or TAVR may lead to risks of coronary obstruction due to the leaflet of the failed valve being pushed up by the new valve and leading to obstruction of blood flow to the coronary arteries. TAVR in failed surgical bioprostheses is common. However, TAVR in failed transcatheter bioprostheses (i.e. transcatheter heart valve-in-transcatheter heart valve) will also become increasingly common. In both situations there is a risk of coronary obstruction. The risk of coronary obstruction can be predicted with the use of cardiac computed tomography. If the predicted risk of coronary occlusion is high, then percutaneous valve-in-valve intervention may be prohibitive. In some cases, the cause of the coronary obstruction is related to the leaflets of the failed surgical or transcatheter heart valve that are pushed up and prevent flow of blood to the coronary arteries.

Transcatheter mitral valve replacement (TMVR) is used to treat mitral valve stenosis and regurgitation. When left untreated, these conditions may cause pulmonary hypertension, heart enlargement, atrial fibrillation, blood clots, and heart failure. TMVR offers a less-invasive alternative to open heart surgery. During TMVR, the mitral valve is replaced with an artificial valve via a catheter. But in many of these procedures, anatomical features of the heart can get in the way. In some cases, the heart leaflet is pushed back and blocks blood flow, causing left ventricular outflow tract (LVOT) obstruction. A transcatheter mitral valve in ring procedure, also known as TMVIR, is when a transcatheter valve is placed in to an existing surgically implanted mitral ring to treat a failing mitral valve repair. The TMVIR procedure is used to treat either a leaky or tight mitral valve that has had a prior placed mitral valve repair with placement of a mitral valve ring. Finally, valve-in-valve (ViV) transcatheter mitral valve replacement (TMVR) is a technique that has emerged as a safe and effective therapeutic option for patients with degenerated mitral valve bioprostheses at high-risk for repeat surgical mitral valve replacement.

### SUMMARY

There is a need for systems, devices, and procedures for excision of portions of a native aortic valve and/or mitral valve or an implanted artificial aortic valve and/or mitral valve. An embodiment of the present disclosure includes a surgical system for excising portions of an aortic valve, a mitral valve, and artificial implanted valves.

Another embodiment is a surgical system with a shaft having a proximal end and a distal end spaced from the proximal end along a central axis. The surgical system includes a capture element carried by the shaft. The capture element has a first expandable portion and a second expandable portion spaced relative to the first expandable portion along the central axis. The first expandable portion and the second expandable portion are configured to expand to seize a portion of a leaflet of the valve. Another embodiment is a surgical system that includes a shaft having a proximal end, a distal end spaced from the proximal end along a central axis, and an outer perimeter that extends around the central axis. The cutting element is carried by the shaft and has a curved cutting edge that extends around a portion of the outer perimeter of the distal end of the shaft. The curved cutting edge is configured to create a curved opening in a leaflet of a valve.

Another embodiment of the present disclosure is a surgical system that has a shaft with a proximal end and a distal end spaced from the proximal end along a central axis. The surgical system includes a piercing element configured to puncture a leaflet of a valve and a capture element. The capture element has a first expandable portion and a second expandable portion spaced relative to the first expandable portion along the central axis. The first expandable portion and the second expandable portion are configured to expand to seize a portion of a leaflet of the valve.

Another embodiment of the present disclosure is a surgical system having a shaft. The shaft has a proximal end, a distal end spaced from the proximal end along a central axis, and an outer perimeter that extends around the central axis. The surgical system includes a piercing element configured to puncture a leaflet of a valve. The surgical system also includes a cutting element carried by the shaft and moveable relative to the piercing element. The cutting element has a curved cutting edge that extends around a portion of the outer perimeter of the distal end of the shaft. The curved cutting edge is configured to create a curved opening in a leaflet of a valve.

Another embodiment of the present disclosure is a surgical system. The surgical system includes a shaft having a proximal end and a distal end spaced from the proximal end along a central axis. The surgical system includes a piercing element configured to puncture a leaflet of a valve. The surgical system also includes a cutting element having a first cutter and a second cutter carried at least partly by the shaft. The first cutter and the second cutter are configured to separate outward away from each other while moving in a proximal direction to cut away a portion of the leaflet while the capture element seizes the leaflet of the valve.

Another embodiment of the present disclosure includes a surgical system. The surgical system includes a shaft having a proximal end, a distal end spaced from the proximal end along a central axis, and an outer perimeter the extends around the central axis. The surgical system includes a capture element having a first expandable portion and a second expandable portion spaced relative to the first expandable portion along the central axis. The first expandable portion and the second expandable portion are configured to expand in order to seize a portion of a leaflet of the valve. The surgical system also includes a cutting element moveable relative to the shaft. The surgical system includes a cutting element having a curved cutting edge that extends around a portion of the outer perimeter of the distal end of the shaft. The curved cutting edge is configured to create a curved opening in a leaflet of a valve.

Another embodiment of the present disclosure is a surgical system. The surgical system includes a shaft having a proximal end and a distal end spaced from the proximal end along a central axis. The surgical system includes a capture element having a first expandable portion and a second expandable portion spaced relative to the first expandable portion along the central axis. The first expandable portion and the second expandable portion are configured to expand in order to seize a portion of a leaflet of the valve. The surgical system also includes a cutting element having a first cutter and a second cutter carried at least partly by the shaft. The first cutter and the second cutter are configured to separate outward away from each other while moving in a proximal direction to cut away a portion of the leaflet while the capture element seizes the leaflet of the valve.

Another embodiment of the present disclosure includes a surgical system. The surgical system has a shaft with a proximal end and a distal end spaced from the proximal end along a central axis. The surgical system includes a first cutting element carried by the shaft. The first cutting element has a curved cutting edge that extends around a portion of the outer perimeter of the distal end of the shaft. The curved cutting edge is configured to create a curved opening in a leaflet of a valve. The surgical system also has a second cutting element having a first cutter and a second cutter carried at least partly by the shaft. The first cutter and the second cutter are configured to separate outward away from each other while moving in a proximal direction to cut away a portion of the leaflet while the capture element seizes the leaflet of the valve.

Another embodiment of the present disclosure is a surgical system. The surgical system has a shaft having a proximal end and a distal end spaced from the proximal end along a central axis. The surgical system has a piercing element configured to puncture a leaflet of a valve. The surgical system has a capture element having a first expandable portion and a second expandable portion spaced relative to the first expandable portion along the central axis. The first expandable portion and the second expandable portion are configured to expand in order to seize a portion of a leaflet of the valve. The surgical system also has a first cutting element carried by the shaft. The first cutting element has a curved cutting edge that extends around a portion of the outer perimeter of the distal end of the shaft. The curved cutting edge is configured to create a curved opening in a leaflet of a valve. The surgical system has a second cutting element having a first cutter and a second cutter carried at least partly by the shaft. The first cutter and the second cutter are configured to separate outward away from each other while moving in a proximal direction to cut away a portion of the leaflet while the capture element seizes the leaflet of the valve.

Another embodiment of the present disclosure is a surgical system. The surgical system includes a shaft having a proximal end and a distal end spaced from the proximal end along a central axis. The surgical system includes a capture element carried by the shaft. The capture element has an expandable portion and fixed collar spaced relative to the expandable portion along the central axis. The expandable portion is configured to expand in order to seize a portion of a leaflet of the valve with the collar.

Furthermore, the shaft in any of the surgical system embodiments disclosed above may include an inner channel that extends from the distal end toward the proximal end. In this regard, the shaft may, but is not required, be one or more catheters.

In another embodiment, in any of the surgical system embodiments disclosed herein, the system also includes a steering element configured to guide the distal end of the shaft toward a target location of a valve.

Another embodiment of the present disclosure is a method for excising a portion of a leaflet of a valve. The method includes advancing a steerable shaft into a cardiovascular system so that its distal end is proximate a valve. The method also includes advancing a piercing element along a central axis of the shaft and into contact with a leaflet of the valve. The method includes piercing, with the piercing element, the leaflet of the valve to form a pierced opening in the leaflet of the valve. The method includes advancing a capture element along the central axis and through the pierced opening in the leaflet. The method also includes deploying the capture element from an insertion configuration into a capturing configuration and transitioning the capture element from the deployed configuration into a capturing configuration to seize the leaflet with the capture element. With the leaflet seized by the capture element, the method forms a curved cut in the leaflet proximate the pierced opening with a first cutting element. The curved cut extends around a portion of the central axis. While seizing the leaflet with the capture element to maintain the relative position of the leaflet relative to the shaft, the method includes inserting a second cutting element having a first and second cutters through the leaflet. The method then includes retracting the second cutting element, while splaying apart the first cutter and a second cutter away from each other, to remove a portion of the leaflet from the valve.

### BRIEF DESCRIPTION OF THE DRAWINGS

The foregoing summary, as well as the following detailed description of illustrative embodiments of the present application, will be better understood when read in conjunction with the appended drawings. For purposes of illustrating the present application, the drawings show exemplary embodiments of the present disclosure. It should be understood, however, that the present disclosure is not limited to the precise arrangements and instrumentalities shown in the drawings. In the drawings:
Figure 1 is a perspective view of a surgical system for excising a portion of an aortic or mitral valve according to an embodiment of the present disclosure;
Figure 2 is a top view of the surgical system illustrated in Figure 1;
Figure 3 is a perspective view of a distal end of the surgical system shown in Figure 1;
Figure 4 is a top plan view of the distal end of the surgical system shown in Figure 3;
Figure 5 illustrates the distal end of the surgical system shown in Figure 1, with a piercing element thereof approaching a leaflet of an aortic or mitral valve;
Figure 6 illustrates the distal end of the surgical system shown in Figure 1, with a piercing element thereof advancing through a leaflet of an aortic or mitral valve;
Figure 7 is a front perspective view of Figure 6, illustrating the distal end of the surgical system shown in Figure 1, with a piercing element thereof advancing through a leaflet of an aortic or mitral valve;
Figure 8 illustrates a capture element of the surgical system shown in Figures 1-7, with the capture element seizing a portion of the leaflet;
Figure 9 illustrates the capture element of the surgical system shown in Figure 8, with the capture element seizing a portion of the leaflet and retraction toward the distal end of the shaft;
Figure 10 illustrates a first cutting element of the surgical system shown in Figures 1-7, forming a curved opening in the leaflet;
Figure 11 is a perspective view of Figure 10, illustrating a first cutting element of the surgical system shown in Figures 1-7, forming a curved opening in the leaflet;
Figure 12 is a top view of a second cutting element of the surgical system shown in Figures 1-11, advancing through the curved opening in the leaflet while the capture element continues to seize the leaflet;
Figures 13 is a perspective view of the second cutting element of the surgical system shown in Figure 12, advancing through the curved opening in the leaflet while the capture element continues to seize the leaflet;
Figure 14 is a top view of a second cutting element of the surgical system shown in Figures 1-11, showing the catheter retracted while the capture element continues to seize the leaflet;
Figure 15 is a perspective view of a second cutting element of the surgical system shown in Figures 1-11, showing the catheter retracted while the capture element continues to seize the leaflet;
Figures 16 is a top view of the second cutting element of the surgical system shown in Figures 1-11, being retracted proximally, and excising a portion of the leaflet while the capture element continues to seize the leaflet;
Figure 17 is a top view of the second cutting element of the surgical system shown in Figures 1-11, being retracted proximally into the catheter and excising a portion of the leaflet while the capture element continues to seize the leaflet;
Figure 18 is a perspective view of the second cutting element of the surgical system shown in Figures 17, shown being retracted proximally and excising a portion of the leaflet while the capture element continues to seize the leaflet;
Figure 19A is a top view of the second cutting element and capture element of the surgical system shown in Figures 1-11, being retracted proximally into the catheter;
Figure 19B is a perspective view of the second cutting element of the surgical system shown in Figure 19A;
Figure 20 illustrates a perspective view of a distal end of the surgical system according to another embodiment of the present disclosure;
Figure 21 illustrates the surgical system shown in Figure 20, with the components thereof advanced distally out of the catheter;
Figure 22 illustrates the first cutting element according to another embodiment;
Figure 23 illustrates the first cutting element shown in Figure 22 according to another embodiment;
Figure 24 is a perspective view the distal end of the surgical system according to another embodiment of the present disclosure;
Figure 25 is a side view of the surgical system shown in Figure 24;
Figure 26 illustrates the surgical system shown in Figure 24 with a positioning element disposed thereon;
Figure 27 illustrates the surgical system according to another embodiment of the present disclosure;
Figure 28 illustrates the surgical system according to another embodiment of the present disclosure;
Figure 29 is a side view of the second cutting element (cutting hooks) according to an embodiment of the present disclosure;
Figure 30 is another side view of the second cutting element shown in Figure 29;
Figure 31 is a perspective view of the second cutting element shown in Figures 29 and 30;
Figure 32 is a perspective view of the second cutting element in a first configuration;
Figure 33 is a perspective view of the second cutting element in a second configuration;
Figure 34 is a perspective view of the cutting elements positioned for engagement with the leaflet;
Figure 35 is a perspective view of the first cutting element advanced to the leaflet and forming a curved opening while the capture element continues to seize the leaflet;
Figure 36 is a top view of the surgical system shown in Figure 35;
Figure 37A illustrates a cutting element of the surgical system according to another embodiment of the present disclosure;
Figure 37B is a side view a cutting assembly configured to sever a mitral chordae tendineae, according to an embodiment of the present disclosure, illustrating a capture element attached to an excised portion of a native mitral valve with intact tendineae;
Figure 37C is a side view a cutting assembly configured to sever a mitral chordae tendineae, according to an embodiment of the present disclosure, illustrating a snare surrounding the tendineae;
Figure 37D is a side view a cutting assembly configured to sever a mitral chordae tendineae, according to an embodiment of the present disclosure, illustrating a snare surrounding the tendineae;
Figure 37E is a side view a cutting assembly configured to sever a mitral chordae tendineae, according to an embodiment of the present disclosure, illustrating severed tendineae;
Figure 37F is a side view a cutting assembly configured to sever a mitral chordae tendineae, according to an embodiment of the present disclosure, illustrating a snare surrounding the tendineae, and retracted into contact with the first cutting element on the catheter;
Figure 38A is a side schematic view of a heart showing placement of various sheaths and guidewires in a cardiovascular system, including a TAVR sheath with a wire (LV wire) extending into the left ventricle and another sheath with another wire (AA wire) extending into the ascending aorta;
Figure 38B illustrates a dilator and/or introducer placed over the LV wire and advanced into the ascending aorta and showing a steerable catheter advanced over the dilator/introducer;
Figure 38C illustrates the steerable catheter steered into position and the dilator/introducer removed;
Figure 38D illustrates an hemoshield catheter advanced over the AA wire;
Figure 38E illustrates deployment of the hemoshield distal to the valve;
Figure 38F illustrates deployment of the hemoshield distal to the valve and expanded outwardly and adjustment of regurgitation as needed;
Figure 38G illustrates deployment of the surgical system through the steerable catheter;
Figure 38H illustrates adjustment of the catheter so that the distal end of the surgical system is in contact with the leaflet of the valve;
Figure 38I illustrates a piercing element puncturing the leaflet of the valve, using radio frequency energy from an electrosurgical unit;
Figure 38J illustrates a capture element advancing into a position and seizing the leaflet;
Figure 38K illustrates the first cutting element advanced to the leaflet and forming a curved opening while the capture element seizes the leaflet;
Figure 38L illustrates the second cutting element (first and second cutting hooks) advancing through the curved opening while the capture element seizes the leaflet;
Figure 38M illustrates the surgical system being retracted slightly so the first and second cutting hooks are able to splay apart while applying tension to the cutters to ensure contact with the leaflet;
Figure 38N illustrates cutting the leaflet portion from the valve;
Figure 38O illustrates cutting the leaflet portion from the valve;
Figure 38P illustrates the surgical system retracting the cutters, capture element and excised portion of leaflet into the catheter;
Figure 38Q illustrates readjustment of the regurgitation via the hemoshield as needed;
Figure 38R illustrates the retraction of the surgical system into the steering catheter; the above illustrated steps can be repeated for each leaflet in the valve;
Figure 39A illustrates a side schematic view of a heart showing the left ventricular outflow tract (LVOT), the mitral valve, and the left ventricle;
Figure 39B illustrates antegrade access of a surgical system, according to another embodiment of the present disclosure, with the steerable catheter positioned superior to the mitral valve in the right atrium;
Figure 39C illustrates advancement of the surgical system through the steerable catheter so that the distal end of the surgical system is in contact with the leaflet of the mitral valve;
Figure 39D illustrates advancement of a piercing element of the surgical system through the leaflet of the mitral valve;
Figures 40A is a side schematic view of a heart showing the LVOT, the mitral valve, and the left ventricle;
Figure 40B illustrates retrograde access of a surgical system, according to another embodiment of the present disclosure, with the steerable catheter positioned inferior to the mitral valve in the left ventricle;
Figure 40C illustrates advancement of the surgical system through the steerable catheter so that the distal end of the surgical system is in contact with the leaflet of the mitral valve; and
Figure 40D illustrates advancement of a piercing element of the surgical system through the leaflet of the mitral valve.

### DETAILED DESCRIPTION OF ILLUSTRATIVE EMBODIMENTS

Systems and methods as set forth in the present disclosure may be used to access, cut, and remove portions of leaflets or other tissue or deposits from an aortic or mitral valve, whether native or implanted.

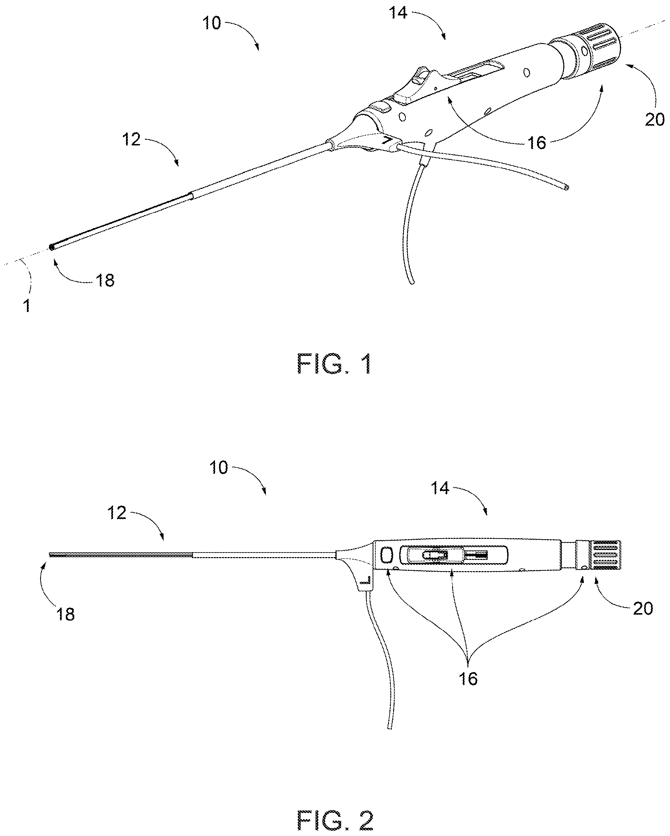

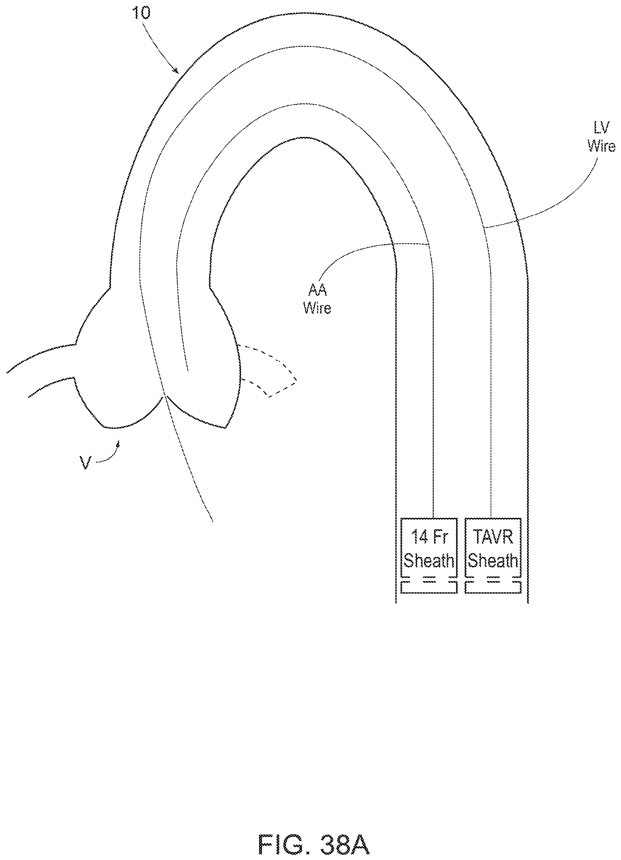

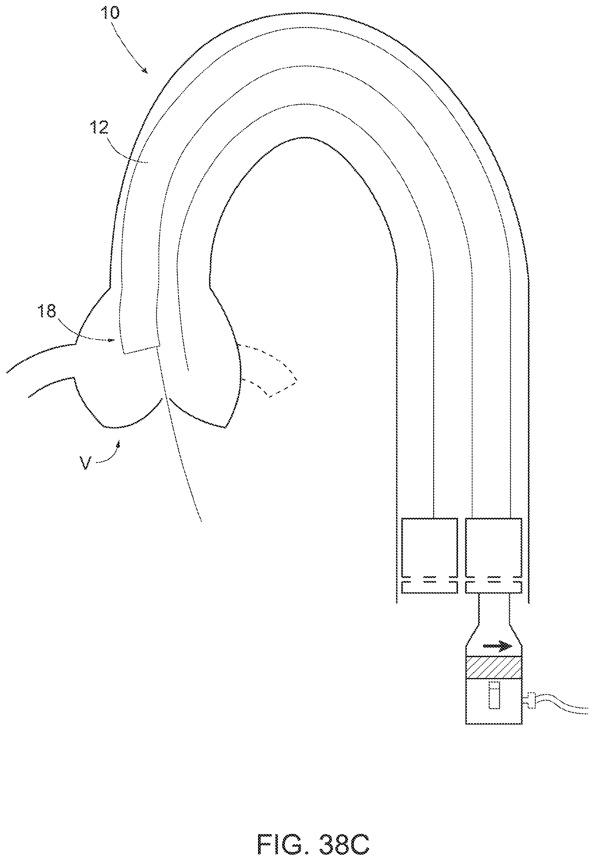

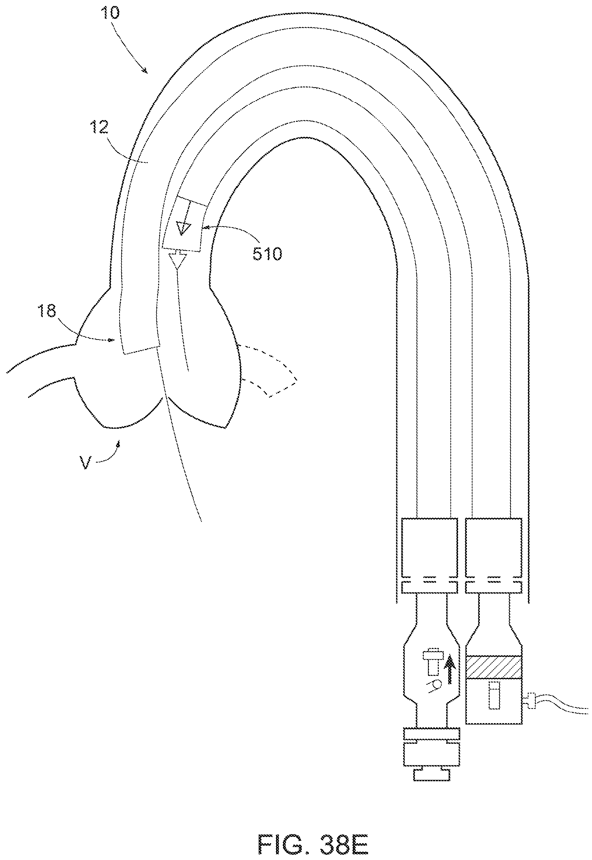

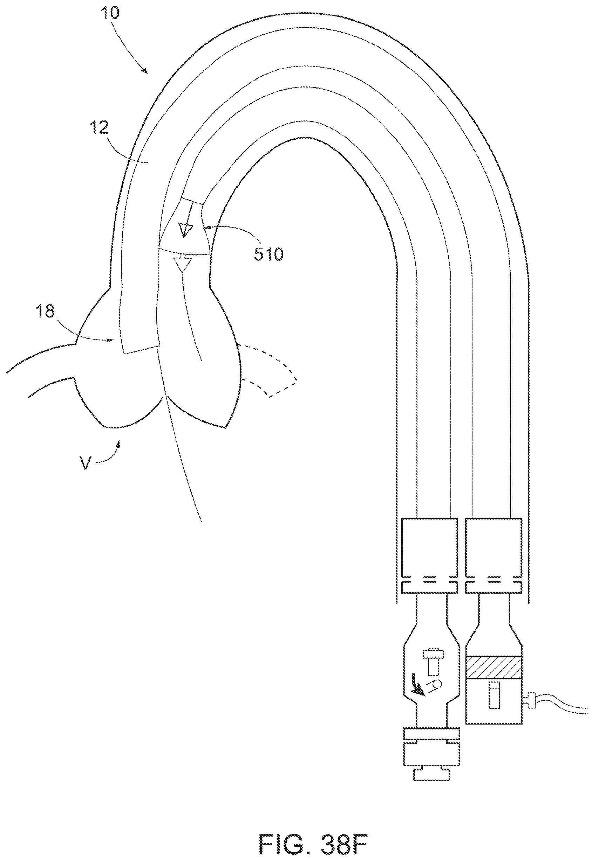

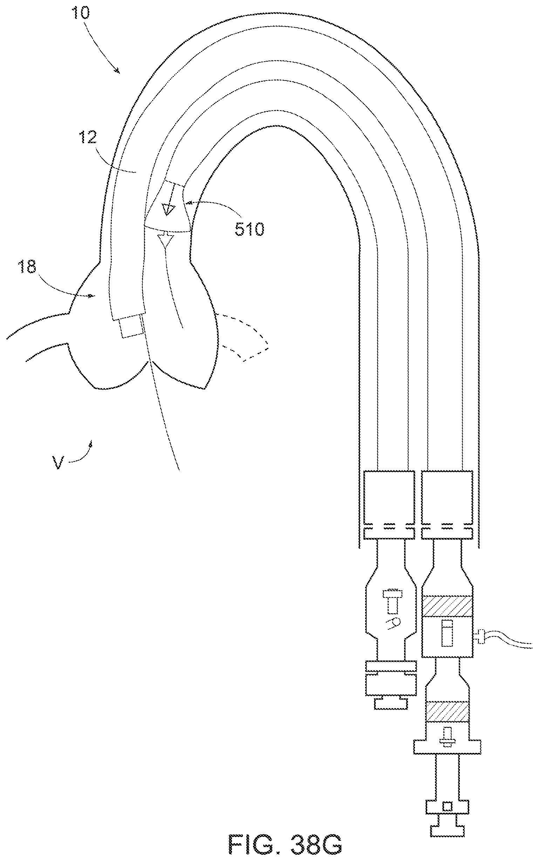

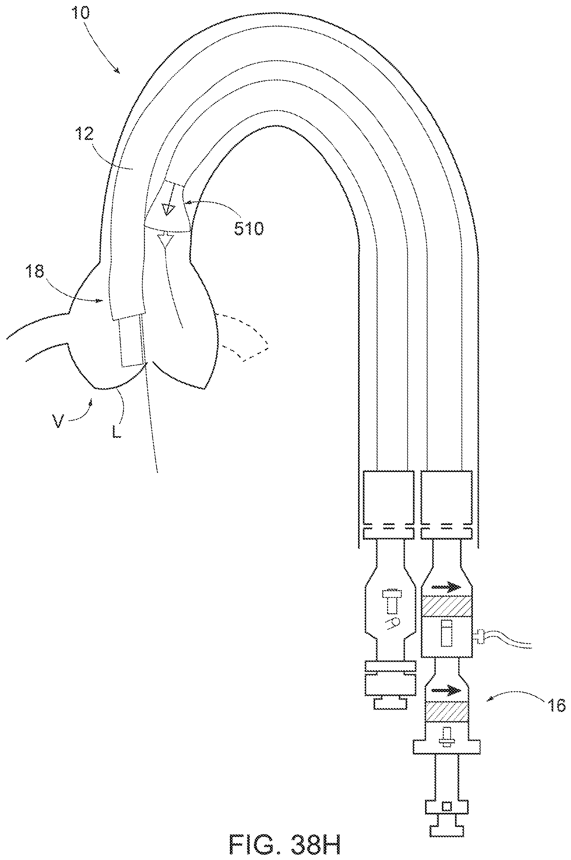

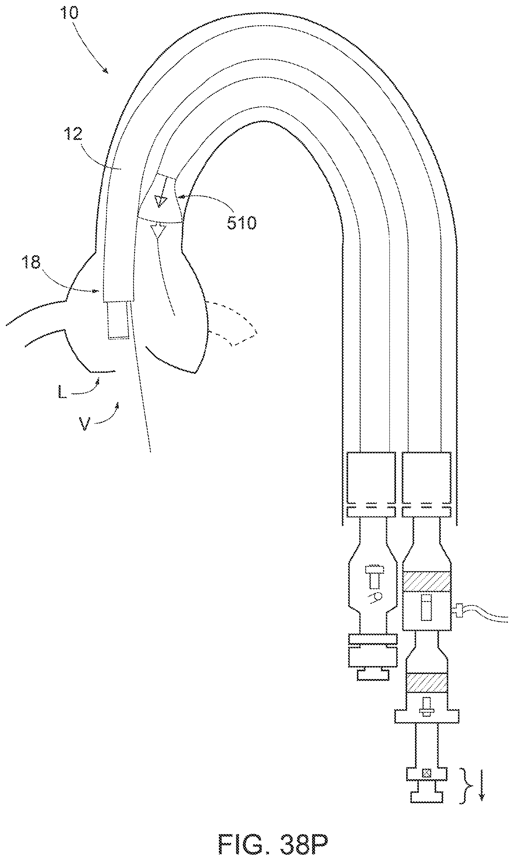

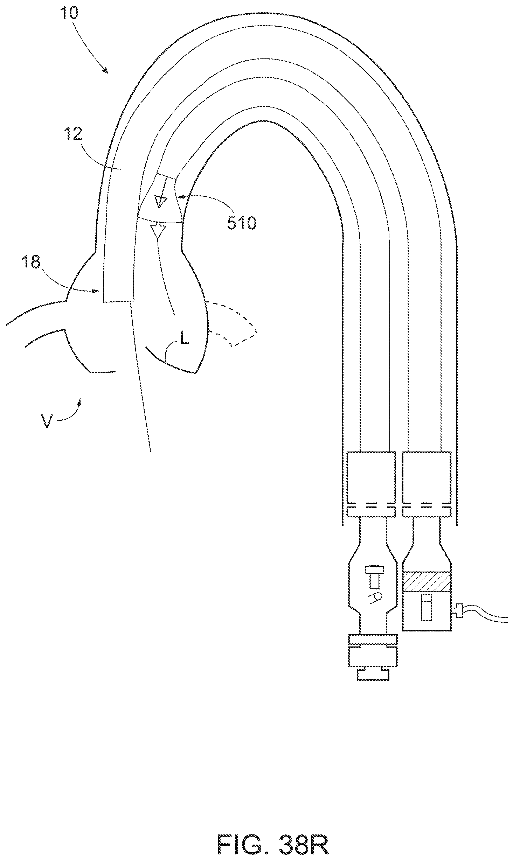

Figures 1-2 illustrates a surgical system 10 used to facilitate the cutting and removal of leaflet portions from a valve of a heart. Figures 38A-38R specifically illustrate an aorta and its aortic arch with the surgical system 10 deployed over the aortic arch and proximate to the aortic valve. However, as further described below, the surgical system 10 as described herein can be used to access and/or treat mitral valves, such as native mitral valves or implanted artificial mitral valves. In Figures 38A-38R, a TAVR sheath, used to access the valve, is shown in the descending aorta. While a system may be used with TAVR, embodiments of the present disclosure may be used with surgical or native valves as well. In embodiments of the present disclosure, an aortic arch may include exemplary TAVR valve (not shown) implanted at the aortic annulus and having damaged leaflets that fail to coapt. Certain systems as described herein can be used to excise damage leaflets.

Continuing with Figures 1 and 2, the system 10 (and related and methods) as described herein are configured to provide access to, and the ability to safely remove, portion of the valve structure. The surgical system 10 may therefore include one or more distinct elements designed to guide the system toward and then puncture, grab or seize, cut, and remove a portion of the leaflet of the valve. More specifically, the surgical system 10 may include one or more of the following elements, either combined in a single assembly or comprising separate modular components, designed to achieve desired leaflet removal: (a) a steering element for targeting the system toward the desire tissue site; (b) a piercing element design to puncture, pierce, or otherwise create an opening in the tissue (e.g. the leaflet); (c) a capture element configured to seize a portion of the leaflet; (d) a first cutting element for creating a curved cut or opening in the tissue while the capture element holds the tissue; and (e) a second cutting element to sever and excise the leaflet while the capture element holds leaflet and help maintain adequate tension. The surgical system may include a handle 14 and may include one or more actuators 16 that are configured to control operation of and relative movement of elements ((a) through (e) above) of the system 10 in use.

The surgical system 10 is generally sized and configured for insertion into a TAVR sheath positioned in the ascending aorta. The system 10 may include additional devices, such as guide wires, introducers, etc., to facilitate introduction of the surgical system into the aortic arch. In terms of size, the distal end 18 and shafts 12 of the system 10 may be sized to fit within a TAVR sheath. For example, the surgical system shaft 12 may have an outer diameter, measured perpendicular to a central axis 1 thereof, up to about 14F. The inner diameter of the introducer (if used) is sized to fit around a guidewire and that may be at least 0.035 inches. However, the inner diameter of the introducer or other components, which could receive a guide wire, may vary. Furthermore, the effective length of the surgical system 10, such as the portion that extends from the entry site of patient to the target location in the aorta may vary. In some examples, the effective length may range between about 40 cm up to about 150 cm, and any intervals therebetween. Accordingly, the surgical system size and configuration could vary as needed.

The surgical system 10, and specifically the one or more elements described above ((a) through (e) and further described below) include elongate shafts 12 that engage or are coupled to the handle 14 and are designed to extend to the target valve, either alone, through a steerable catheter, and/or through a procedural sheath, such as a TAVR sheath. Some or all of the elongate shafts 12 of the elements ((a) through (e)) may be in the form catheters, which include an internal channel through which other devices and elements or may pass. Its form as a catheter is not strictly required but would be useful, as needed, when coupled with other surgical devices for access to and engagement with an implanted valve in the aorta.

As illustrated in Figures 1 and 2, the surgical system 10 includes a distal end 18 and a proximal end 20 that are spaced apart from each other along a central axis 1. The system 10, and the elongate shafts 12 are elongated along the central axis 1. Generally, a direction from the proximal end 20 toward the distal end 18 is referred to herein as the distal direction. A direction from the distal end 18 toward the proximal end 20 is referred to herein as the proximal direction (opposite the distal direction).

The surgical system 10 may include a steering element (not numbered). The steering element may be designed to be inserted through the TAVR sheath and target the distal end 18 of the system 10 toward the valve structure. The steering element may include a shaft with a distal end, a proximal end spaced from the distal end in a proximal direction along a central axis 1. In an alternative embodiment, the steering element, when in the form of a catheter, may also include an inner channel that extends between the distal and proximal ends thereof. A catheter is not strictly required to effectuate steering or targeting as described herein. The steering element is configured to present the distal end of the system 10 into the ascending aorta proximate the valve so that the piercing element, the capture element, and first and second cutting elements, can be actuated as needed to accomplish their respective functional objectives. In such an example, the distal end of the steering element, for example the distal end 18 of shaft 12, can be steered or guided into position as needed to present the elements proximate the valve.

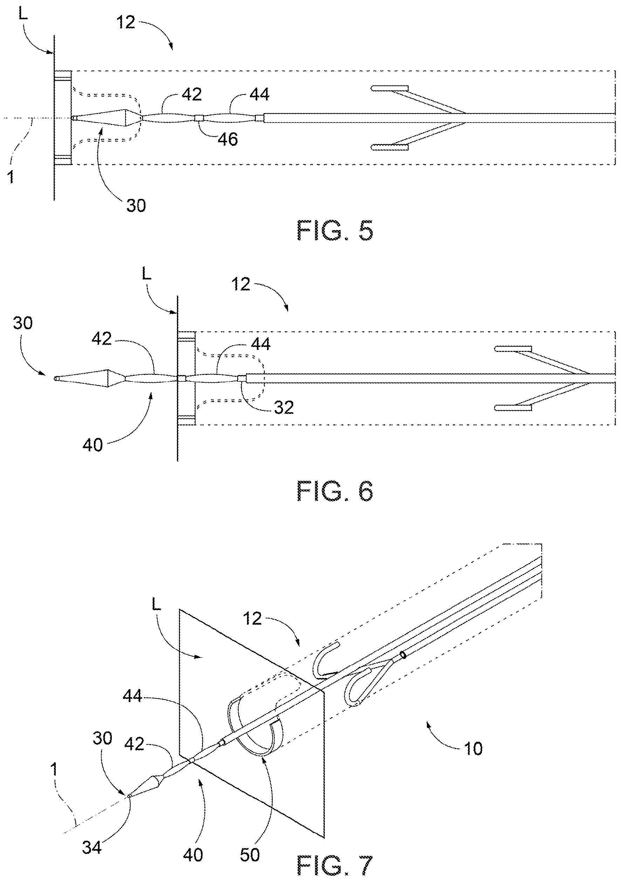

The surgical system 10 may include a piercing element 30. The piercing element 30 may include a shaft 32 having a proximal end and a distal end (proximal and distal ends not numbered) spaced from the proximal end along a central axis 1 and an element at a distal tip 34 that is configured to puncture a leaflet of a valve. The piercing element 30 is movable between a retracted configuration (Fig. 5), where the distal tip 34 of piercing element 30 is located in the inner channel (not numbered) of the shaft 12, and a deployed configuration (Fig. 7) where the distal tip 34 of the piercing element 30 is located outside of the inner channel of the shaft 12. At least one actuator 16 coupled to the handle 14 may be used to move the piercing element 30 between the retracted configuration and the deployed configuration. In one example, a distal tip 34 of piercing element 30 is a sharp tip. In yet another example, distal tip 34 of piercing element 34 has a circular cross-section with sharp leading edge. In another, one, or all of these examples, the distal tip 34 of piercing element 30 is an electrode coupled to an RF unit.

The piercing element 30 when configured as an electrode may be made of stainless steel or nitinol. Furthermore, the piercing element 30 may also include an insulative coating. The piercing element 30 may also include denuded surfaces. Both insulated and denuded surfaces may ensure that energy is directed to the correct places in use.

Referring to Figures 5 through 9, the surgical system 10 may include a shaft (not numbered) having a proximal end and a distal end spaced from the proximal end along the central axis 1 and a capture element 40. The capture element 40 is carried by the shaft and has a first expandable portion 42 and a second expandable portion 44 spaced relative to the first expandable portion 42 along the central axis 1. The first expandable portion 42 and the second expandable portion 44 are configured to expand in order to seize a portion of a leaflet L of the valve. The first expandable portion may be referred to a distal expandable portion and the second expandable portion may be referred to as a proximal expandable portion as needed for clarity.

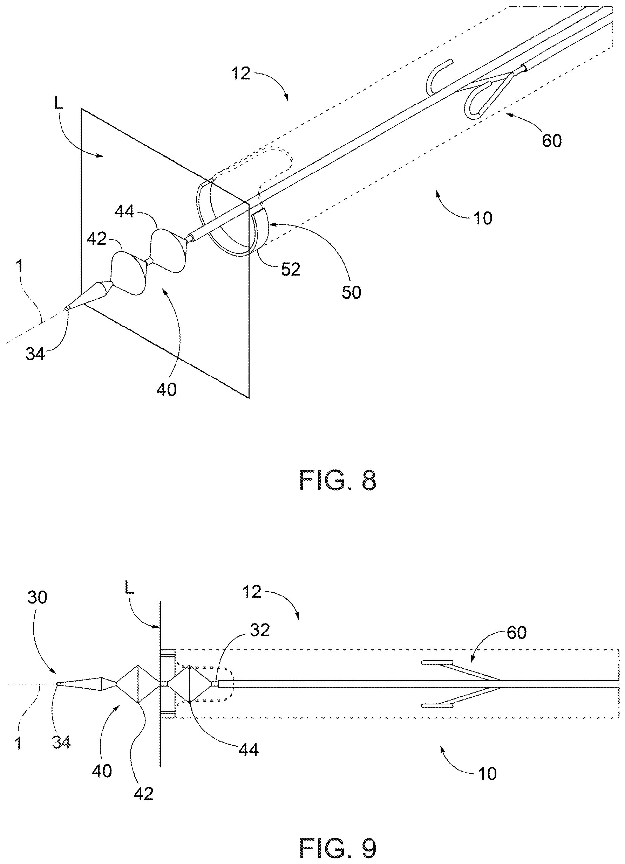

As shown in Figures 5-9, the capture element 40 is configured to seize the leaflet L and stabilize its position for subsequent excision. The capture element 40 has an insertion configuration, as shown in Figures 5-7, where the first expandable portion 42 and the second expandable portion 44 are collapsed toward the central axis 1. The capture element 40, as shown in Figures 8 and 9, also has an expanded configuration where the first expandable portion 42 and the second expandable portion 44 are expanded outwardly away from the central axis 1. Continuing with Figures 8 and 9, the capture element 40 also has a captured configuration, where at least a portion the first expandable portion 42 and at least a portion of the second expandable portion 44 are positioned to seize a portion of the leaflet L while in the expanded configuration. In this example, some portion of the first and second expandable portions can move in a manner sufficient to seize, or grab, the leaflet L. One or more actuators 16 are disposed on the handle 14 (See Figure 1) to control transition of the capture element 40 between and among the insertion configuration, the expanded configuration, and the captured configuration. More specifically, at least one actuator 16 coupled the capture element 40 is configured to control operation of the capture element 40 to seize the leaflet.

The first and second expandable portions 42, 44 may be configured to expand simultaneously. Alternatively, the first and second expandable portions 42, 44 may be configured to expand sequentially. In one example of sequential expansion, the distal expandable portion 42 is actuated first, after which tension is applied to the shaft in the proximal direction to ensure adequate positioning of the capture element 40 relative to the leaflet, followed by expansion of the proximal expandable portion 44 to capture the leaflet. In another example of sequential expansion, the proximal expandable portion 44 is actuated first after which tension is applied to the shaft in the distal direction to ensure adequate positioning relative to the leaflet, followed by expansion of the distal expandable portion 42 to capture the leaflet L.

The first and second expandable portions 42, 44 can be any element, device, structure, or material that is configured to aid in capturing a leaflet L between the first and second expandable portions. In one example, the capture element 40 includes an expandable braid and a collar 46 (Figure 5) disposed along the expandable braid. The collar 46 is configured to separate the expandable braid into the first expandable portion 42 and the second expandable portion 44. In such an example, the expandable braid is a nitinol braid. In yet another example (not shown), the first expandable portion 42 and the second expandable portion 44 are first and second wire portions, respectively, that extends around a shaft. In such an example, the first and second wire portions are configured to expand radially outward. In yet another example, the first expandable portion 42 and the second expandable portion 44 are first and second sets of longitudinal wires respectively, wherein the first and second set of longitudinal wires configured to fold outwardly. In yet another example, the first expandable portion 42 and the second expandable portion 44 is a first balloon and a second balloon, respectively, and a collar 46 is located between the first balloon and the second balloon.

Referring to Figures 8-11, the surgical system 10 includes a cutting element 50 configured to form a curved opening in a leaflet L of the valve. The cutting element 50 may be referred to herein as a first cutting element 50. In this example, the first cutting element 50 is a cutting element carried by a shaft of the surgical system 1. As shown, the cutting element 50 has a curved cutting edge 52 that extends around a portion of the outer perimeter of the distal end 12 of the shaft. Configured this way, the curved cutting edge can create a curved opening in a leaflet L of a valve. More specifically, the curved cutting edge of the cutting element has a C-shape such that it does not extend around an entirety of the central axis 1. In use, a curved opening through which the system 10 can partially extend through to facilitate placement of additional cutting elements is described below. In addition, this cutting element 50 allows the capture element 40 to maintain its grip in the leaflet L. The curved cutting edge 52 of the cutting element is a sharp element. In another example, the curved cutting edge 52 is an electrode that is responsive to electrical energy supplied by a separate electrosurgical unit. In this description, electrical energy and RF energy are used interchangeably. The cutting element 50, when configured as electrodes, may be made of stainless steel or nitinol. Furthermore, the cutting element 50 may also include an insulative coating. The cutting element 50 may also include denuded surfaces. Both insulated and denuded surfaces may ensure that energy is directed to the correct places in use.

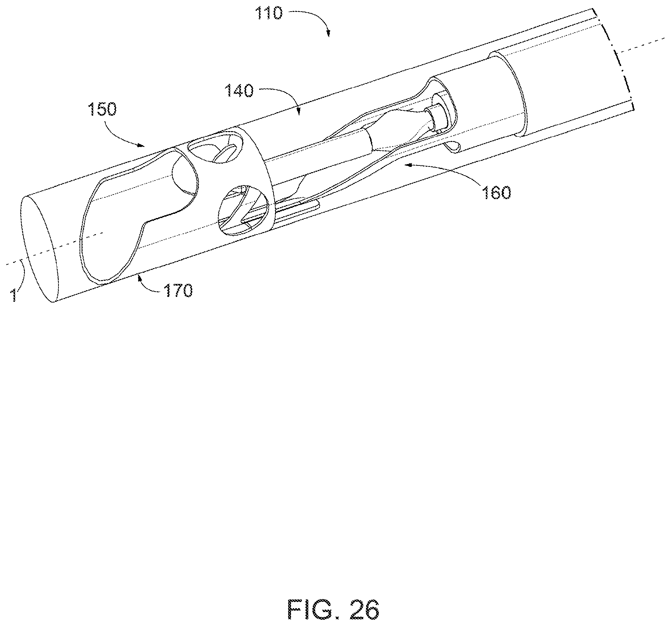

As shown in Figures 9-11, the cutting element 50 forms a part of the distal-most end of the shaft 12. However, the cutting element 50 may be disposed along a separate shaft or a catheter and inserted or moveable through and relative to the shaft 12, as depicted in Figure 26. Like other elements described in this present application, the cutting element may not be carried by a catheter per se. However, a shaft as used herein can include a catheter as described elsewhere in the present disclosure. The separate outer shaft may be configured to aid in the delivery and/or containment of a non-ionic solution, such as dextrose, to displace blood during electrification and concentrate electrical energy on the target leaflet.

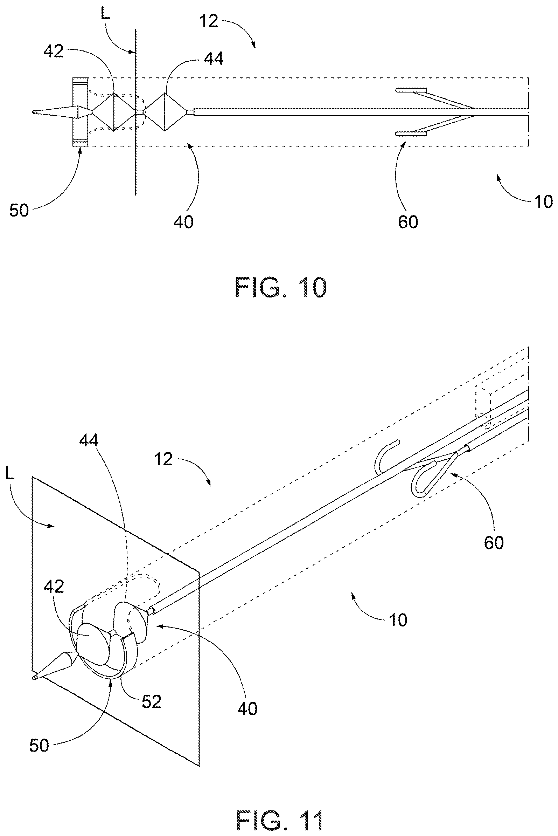

Turning to Figures 10-19B, the surgical system 10 also includes an additional cutting element 60, referred to herein as the second cutting element 60 or an excision cutting element 60. The second cutting element 60 has a first cutter and a second cutter carried at least partly by the shaft. In the illustrated embodiment, the first cutter and the second cutter are cutting hooks. As shown, the shaft (not numbered) may be a separate elongate shaft configured to carry the second cutting element 60 inside the shaft 12. As will be further described below, the first cutter and the second cutter are configured to, in use, separate outwardly away from each other (splay) while moving in a proximal direction to cut away a portion of the leaflet L while the capture element 40 seizes the leaflet L of the valve. In the example, shown, the first cutter and the second cutter each include a) an elongate shaft and b) a cutting hook that extends from the elongate shaft. The cutting hook has a curved body and at least one cutting edge. In general, the cutting edge may be on any location about the circumferential surface of the cutting hooks. In particular, the cutting edge may be on the leading edge, the trailing edge, sides, or any combination thereof. Furthermore, the first and second cutters may include electrodes that are responsive to electrical energy. As such, in one example, an electrosurgical unit may be used to supply electrical energy the electrodes the cutting element 60 during use. The first and second cutters or hooks themselves may serve as or include first and second electrodes. When the first and second cutters are configured as electrodes they may be made of stainless steel or nitinol. Furthermore, these cutters, e.g. hooks, may also include an insulative coating. The first and second cutters may also include denuded surfaces. Both insulated and denuded surfaces may ensure that energy is directed to the correct places in use.

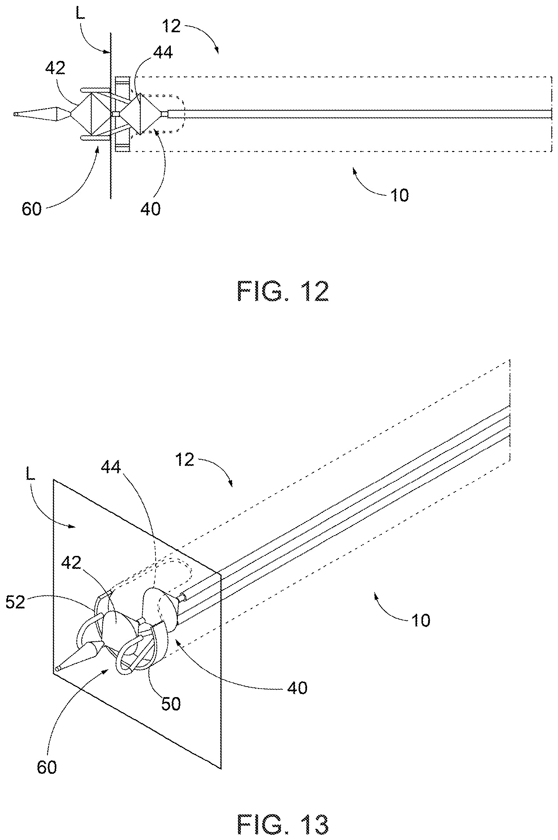

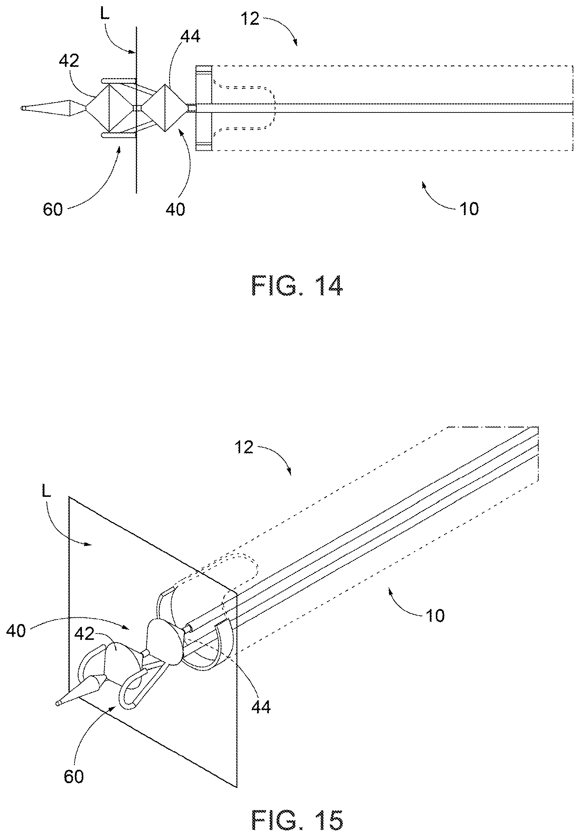

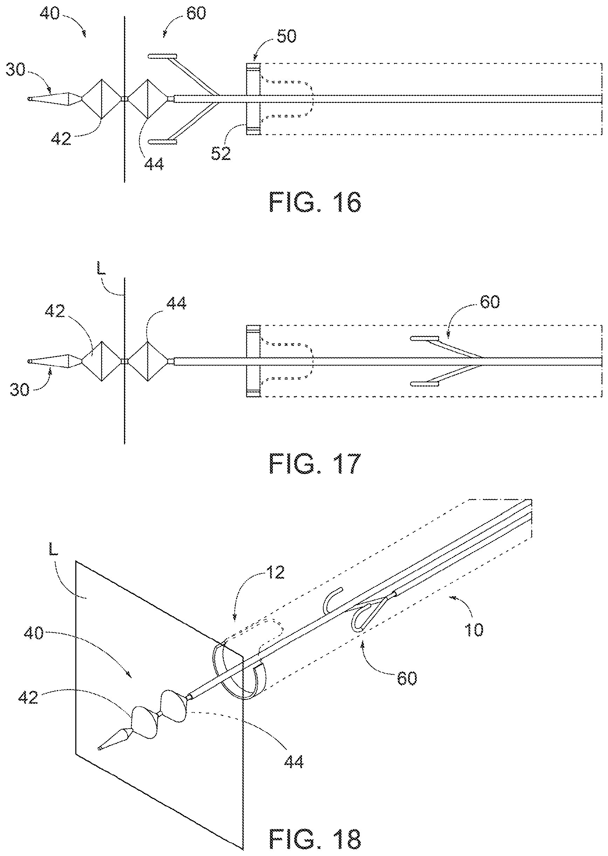

Continuing with Figures 10-19B, in use, the first cutter and the second cutter have an insertion configuration, as shown in Figures 10 and 11, where terminal ends of the first cutter and the second cutter are located inside the inner channel of shaft 12. As shown in Figures 12-15, the first and second cutters have a deployed configuration, where terminal ends of the first cutter and the second cutter is located outside of the inner channel of the shaft 12 and pass through the curved opening created by the first cutting element 50. As shown in Figures 14-15, when the terminal ends of the first and second cutters pass through the opening, the first and second cutters can transition into a cutting configuration, where the terminal ends of the first cutter and the second cutter splay apart with respect to each other. The size, or width of the curved opening, e.g. the c-cut, may inhibit the amount of splay until the second cutting elements are retracted, at which time they can splay further apart, as depicted in Figure 16. Then, as shown in Figures 17-19B, the capture element 40 and the second cutting element 60 are retracted back into the shaft 12 in a proximal direction, taking with it the excised portion of the leaflet. In other embodiments, however, the leaflet remains outside the catheter body and is retracted and removed from the patient via the steerable catheter.

The surgical system may include at least one actuator, located on the handle, which is configured to cause the cutting element 60 to transition through the insertion configuration, the deployed configuration, and the cutting configuration.

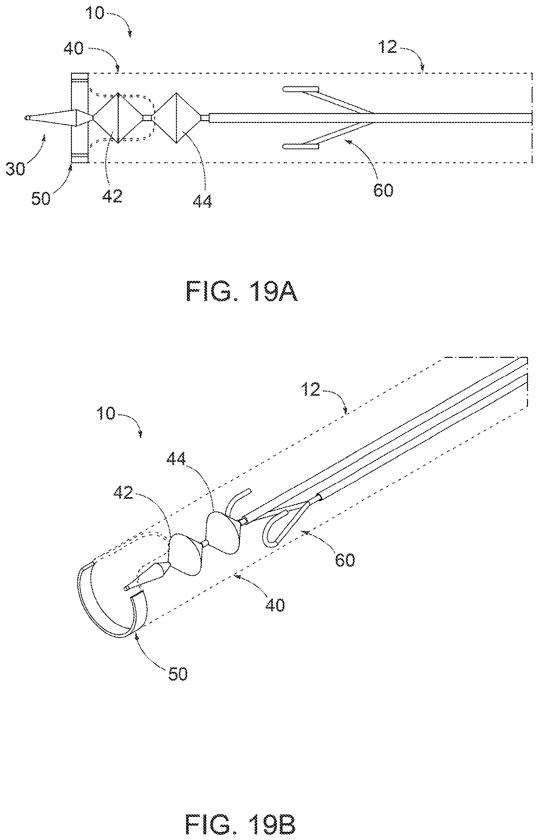

Continuing with Figures 10-19B, the cutting element 60 and capture element 40 may work in concert to excise the leaflet portion. More specifically, while the first and second cutters are in the cutting configuration and moving in the proximal direction, the capture element 40 maintains its relative position while still grabbing the leaflet L. By placing some distally directed force on the capture element 40, tension is maintained in the leaflet, allowing the first and second cutters to splay apart, either through shape memory or by following the natural anatomy of the valve. The first and second cutters separate and splay while moving in the proximal direction, excising a portion of the leaflet L (excised portion not shown). By holding the leaflet L in place while splaying the first and second cutters, the amount of leaflet that can be removed from the valve may be optimized. In other words, holding the leaflet while splaying the cutters to excise the leaflet can maximize the amount of tissue that can be removed from a particular leaflet. In this regard, the excised portion of the leaflet remains attached to the capturing element for subsequent removal from the patient as depicted in Figures 19A and 19B.

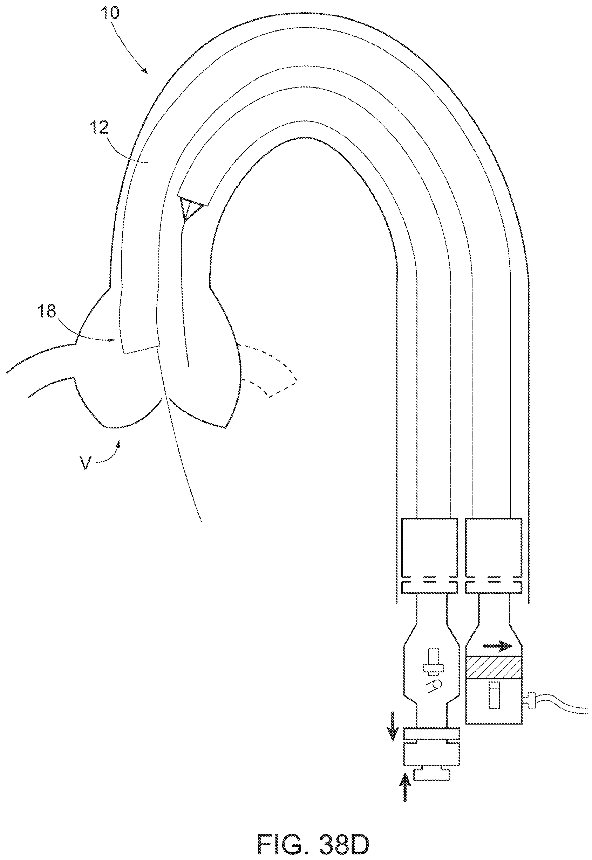

As described above, the piercing element 30, the first cutting element 50 and the second cutting elements 60 may use electrical energy, e.g., via an RF unit, to cut the leaflet as needed. Use of RF energy may cause tissues, blood, and water, etc., to vaporize. This, in turn, may cause formation of bubbles and other emboli that may need to be extracted or removed from the aorta. More specifically, what is generated in the aorta responsive to RF cuts may likely be a combination of water vapor, char, smoke, oxygen, nitrogen, carbon dioxide, solid emboli, such a tissue fragments, etc. Vaporizing tissue and arcing through blood may liberate all of these components, which could indicate a need to manage the capture and removal, i.e., extraction of these components, e.g. via a hemoshield element. The hemoshield element (shown in Figures 38D-38R) may operate as a temporary valve and/or filter to maintain proper blood flow while also capturing debris as needed. In one example, the hemoshield element may be a funnel or trumpet shaped structure that is self-expanded when deployed proximate the valve. It can be positioned to appose the aortic wall in a manner that captures particles from the forward flow ejection of the left ventricle (LV). In one example, the hemoshield element has a curved element on its outer perimeter that facilitates the apposition to the aortic wall and the shaft of the sheath and to the subsequent TAVR sheath. The hemoshield element is further configured to enable adequate forward flow with the LV ejection of blood flow, by opening and closing in response to flow. In some examples, the hemoshield may have (semilunar) overlapping elements in the funnel that close during LV ejection of blood to the aorta and open in diastole. However, semilunar openings are not required. Where openings are present the hemosheid element, these are positioned and designed to allow diastolic flow in the direction of the coronary ostium.

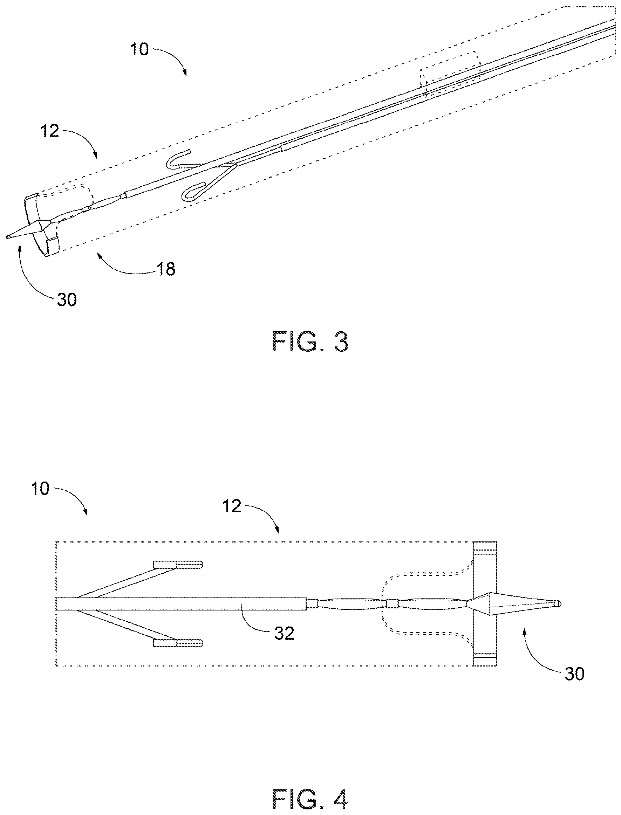

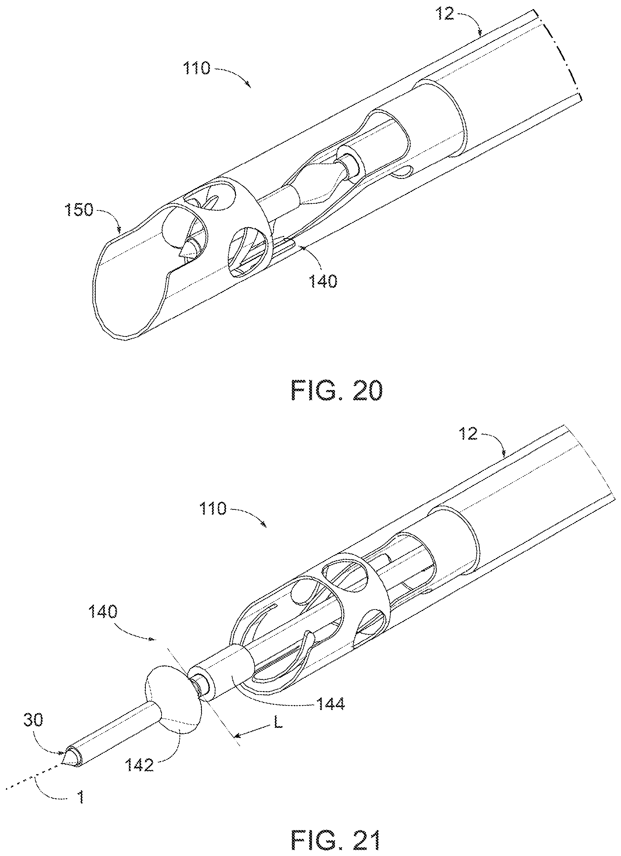

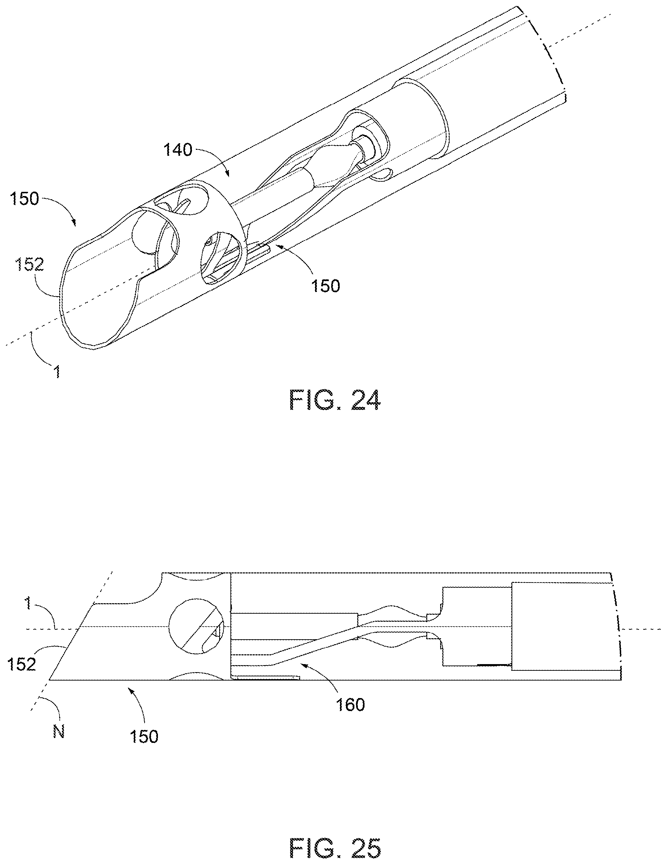

Another embodiment of the surgical system is shown in Figures 20 through 25. As shown, the surgical system 110 shown in Figures 20-25 includes features that are similar to and common with features of surgical system 10 shown in Figures 1-19B. Accordingly, common reference numbers are used to identify features that are common to the surgical system 10 and surgical system 110. More specifically, the surgical system 110 may include one or more of the following elements, either combined in a single assembly or comprising separate modular components, designed to achieve desired leaflet removal: (a) a steering element for targeting the system toward the desire tissue site; (b) a piercing element design to puncture, pierce, or otherwise create an opening in the tissue (e.g. the leaflet); (c) a capture element configured to seize a portion of the leaflet; (d) a first cutting element for creating a curved cut or opening in the tissue while the capture element holds the tissue; and (e) a second cutting element to sever and excise the leaflet while the capture element holds leaflet and help maintain adequate tension. The surgical system 110 may include a handle 14 and may include one or more actuators 16 that are configured to control operation of and relative movement of elements ((a) through (e) above) of the system 10 in use.

In the embodiment shown in Figures 20 and 21, the capture element 140 may include an expandable portion 142 and a fixed collar 144. That is, the proximal portion of the capture element 140 is in the form of a fixed collar 144, as depicted in Figures 20 and 21, and is spaced relative to the distal expandable portion 142 along the central axis 1. In this example, the diameter of the proximal portion, e.g. the fixed collar, is sufficient to prevent traversal of the leaflet through the hole created by the piercing element 30, and sufficient to securely capture the leaflet upon actuation of the distal expandable portion 142. Thus, the expandable portion 142 and fixed collar 144 can seize a portion of the leaflet.

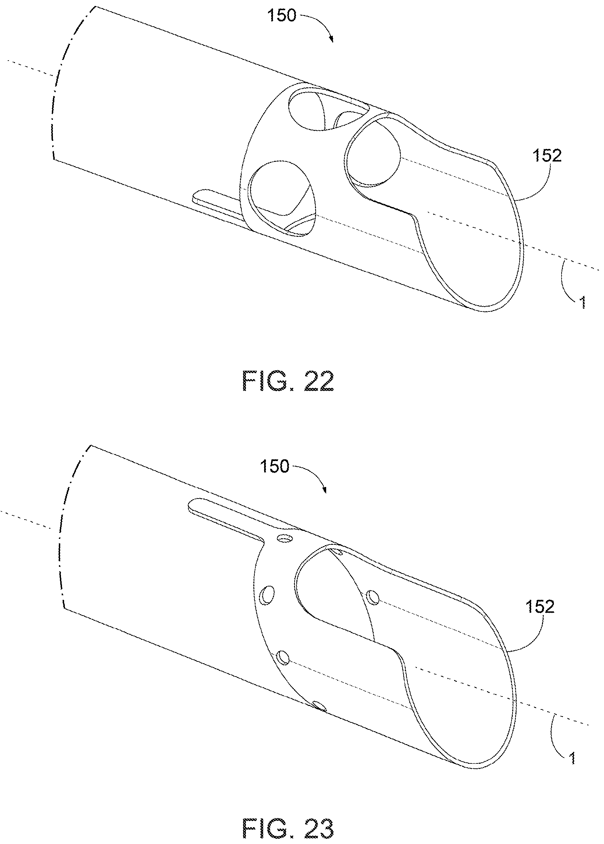

As shown in Figures 22-25, the surgical system 110 may include a cutting element 150 configured to better adapt to leaflet anatomy. In particular, the cutting element 150 may be shaped in a manner to improve contact and positional stability on the target leaflet prior to and during cutting, enhance bond strength with the surgical system shaft, improve flexibility, and optimize radiopacity (visibility and orientation). In such an example the cutting element 150 includes a cutting edge 152 that both curves around a central axis 1 as shown and is also angled with respect to the central axis 1. More specifically, a forward end of the curved cutting edge 152 is angled and lies along a plane N that intersects and is angled with respect to the central axis 1, while also curving around a portion of the central axis 1. In such an example, the curved cutting edge 152 is configured to form a curved cut the leaflet, like a C-shaped cut.

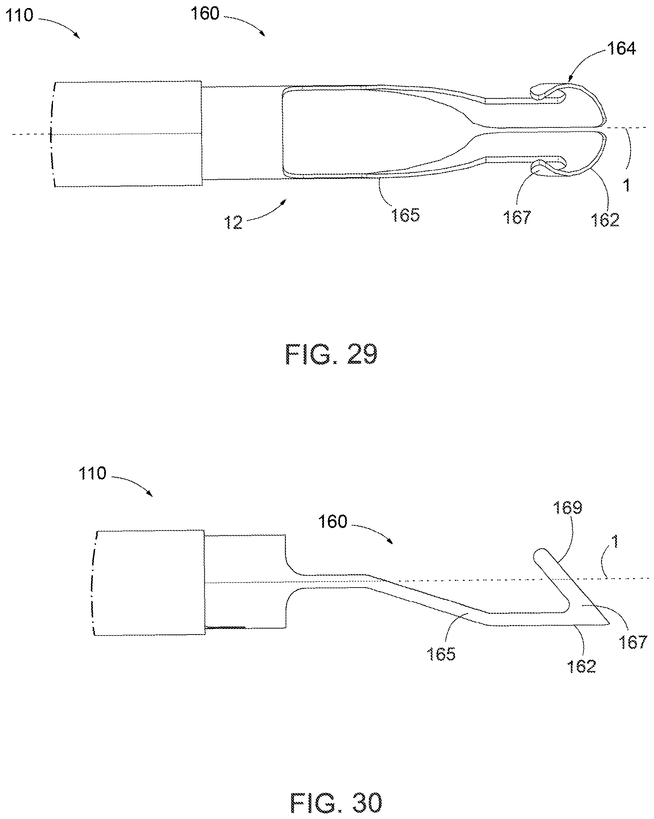

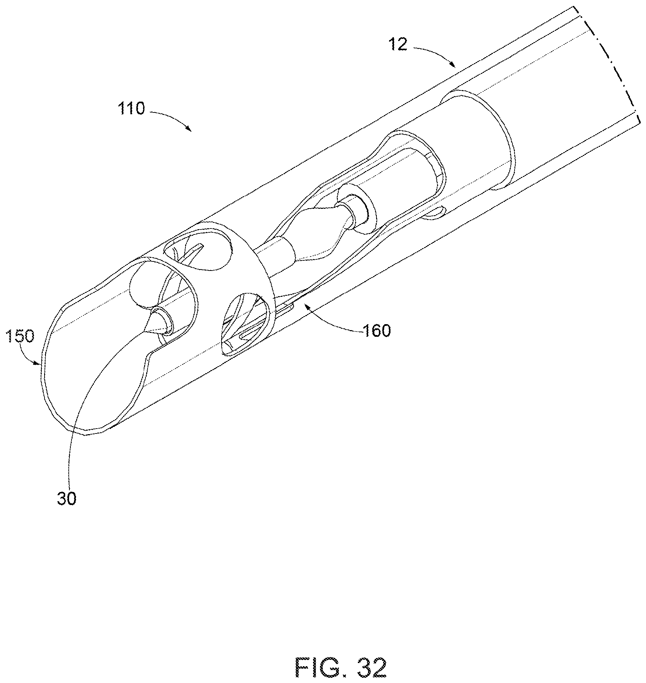

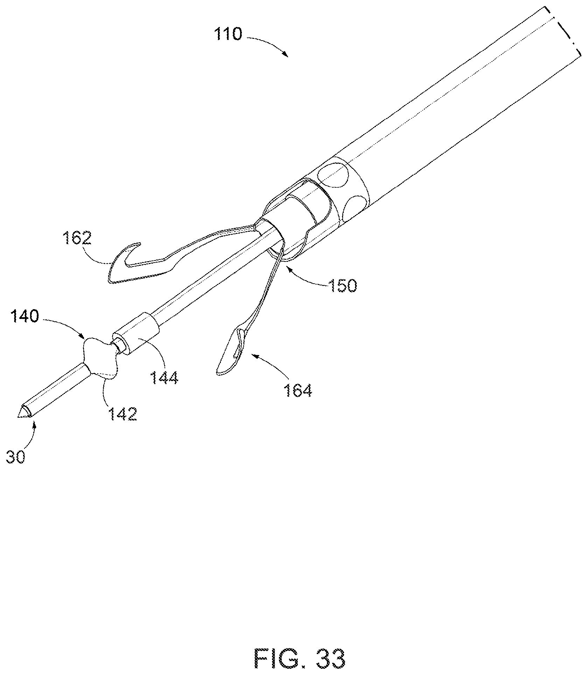

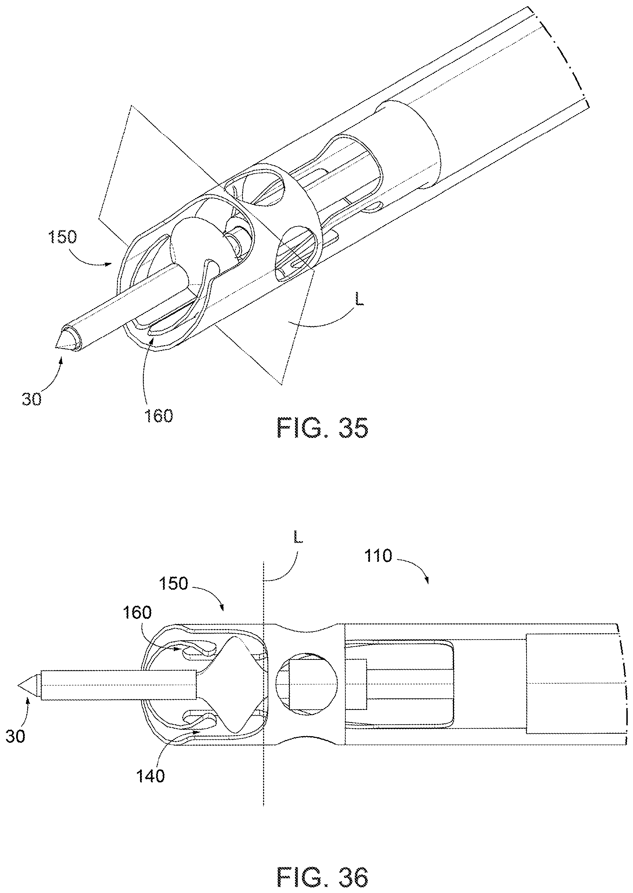

As depicted in Figures 29-35, the surgical system may include a second cutting element 160. The second cutting element 160 may include a first and second cutters 162, 164 designed to sever the leaflet, as describe above with respect to cutting element 60. As best shown in Figures 29-31, the first and second cutters 162, 164 each include a leg portion 165 coupled to the shaft, and a hook portion 167 that extends rearwardly with respect to the leg portion while also curving around the axis 1. The hook portion 167 includes a forward edge 169 that is angled with respect to the axis 1. The forward edge 169 is both angled with respect to the axis 1 and curved around the axis 1. The first and second cutters may be formed by cutting out the shape of the cutters from a shape memory tubular material.

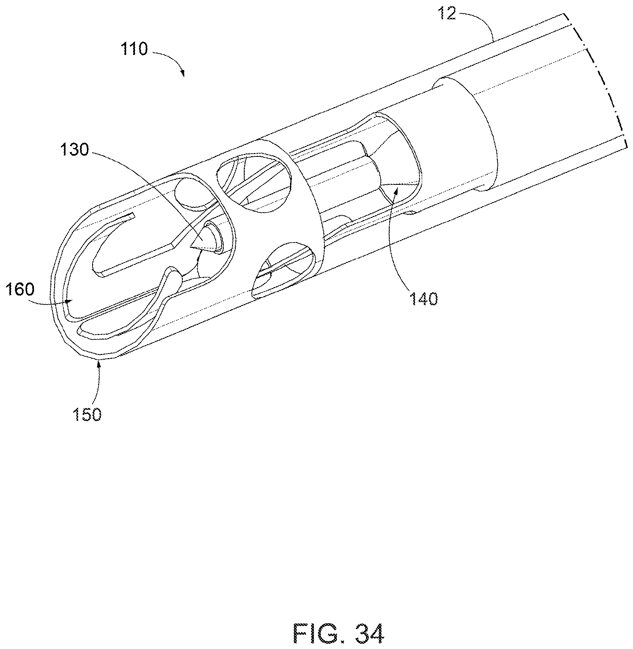

As shown in Figures 34-36, the second cutting element 160 may be positioned in a nested configuration within the first cutting element 150. The orientation permits two modes of operation. In a fist mode of operation, the first cutting element and the second cutting element are configured to advance in tandem, and in a second mode of operation, the second cutting element is configured to advance relative to the first cutting element and the shaft. Thus, allowing the second cutting element 160 cutters to pass through the curved opening in the leaflet created by the first cutting element 150 simultaneously with the first cutting element 150. That is, the first and second cutting elements 150, 160 can, in tandem, advance through the cut opening in the leaflet as the first cutting element creates the curved cut. Upon retraction of only the first cutting element 150 from the leaflet, the two cutters 162, 164 of the second cutting element 160 may splay apart and engage with the leaflet.

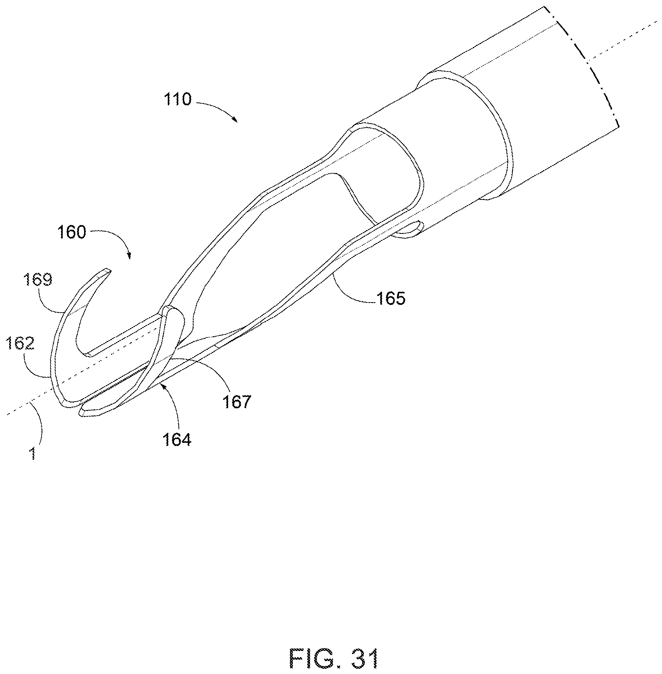

The second cutting element 160 (or 60) cutters may be formed from mandrels comprised of a radiopaque shape memory material. As shown in Figures 31 through 34, the cutters 162, 164 of the second cutting element 160 may also be formed from a hypotube comprised of a radiopaque shape memory material. Cutters formed from a hypotube may improve coaxial positioning and orientation of the second cutting element 160 cutters relative to the other elements contained within the shaft 12 or catheter. Second cutting element 160 cutters formed from a hypotube may also improve re-capturability of the cutters 160 when retracted back into the shaft following leaflet excision.

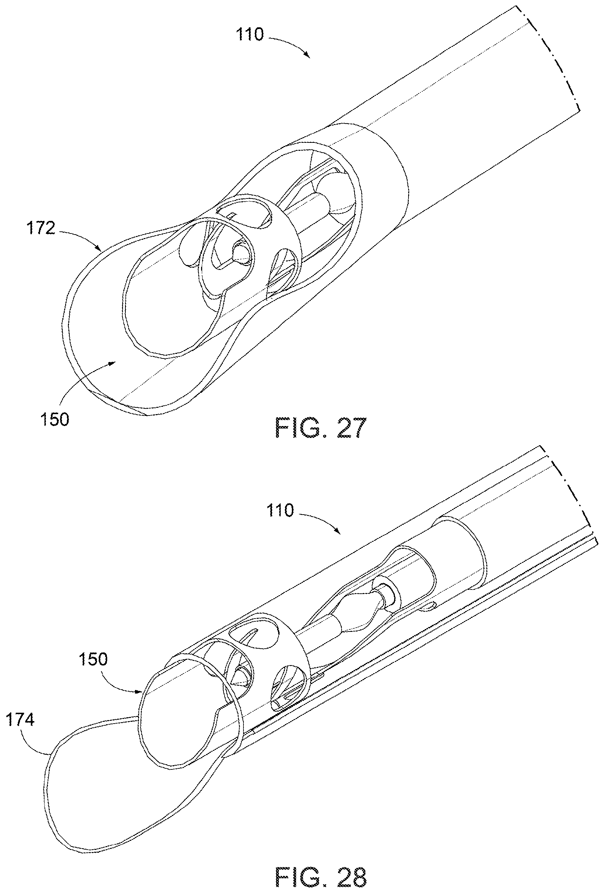

Referring back to Figures 26-28, the shaft 12 may also include positioning elements 170 to improve positioning and positional stability of a distal end of the shaft relative to the tissue, e.g. the target leaflet during a leaflet excision procedure. The positioning elements 170 may be in the form of a slidable tube, a hood, and a loop. Figure 26 illustrates a slidable tube carried by the distal end of the shaft 12. Figure 27 depicts a positioning element in the form of a hood 172 on the distal end of the shaft. The hood 172 may be positioned in the target leaflet nadir to stabilize the surgical system 10, 110 during the procedure. The positioning hood 172 may be cut, molded, heat formed, or affixed to the outer shaft 12. The positioning hood 172 may comprise a radiopaque material or added radiopaque feature. The positioning hood 172 may flex outwardly slightly relative to the shaft 12 to aid in positioning. In another example, as shown in Figure 28, the positioning element is configured in form of a wire positioning loop 174 carried by the distal end of the shaft. The positioning loop 174 that may be positioned in the target leaflet nadir to stabilize the surgical system during the procedure. The positioning loop 174 may be comprised of a radiopaque shape memory material. The positioning loop 174 may be adjustable, i.e., the effective length of the loop can be changed to change the size, shape, or positional orientation of the positioning loop. The positioning loop can be adjusted via an actuator coupled to the handle. Alternatively, the positioning hood or positioning loop may be incorporated into a separate steering element (steerable catheter).

The described elements are shown as separate components, one for capturing the leaflet, one for cutting the leaflet and one for removing the cut leaflet form the aorta. However, it is possible that the elements described herein may be combined into a single surgical system 10, 110 that target, pierce, seize, cut, and remove a leaflet. In one example, the surgical system 10, 110 may be inserted into the implanted TAVR sheath as a single unit. In such an example, the handle of the system 10, 110 is configured to facilitate the control of the various components and subcomponents, via actuators and the like, as described above.

One or more of the elongate shafts of the piercing element, the capture element, the first cutting element, and/or the second cutting element could be in the form of catheters. For example, an outer shaft as shown in Figures 1 and 2 may be a catheter with an inner channel that includes features to carry and guide elongate shafts of the piercing element, capture element, and/or the first and second cutting elements. In some case, the capture element may be a catheter through which the piercing element and its shaft is carried and can travel toward the leaflet. In such an example, the first and second cutting elements can be carried around and along the capture element.

The shafts described herein, when in the form of catheters, will generally include a shaft, an inner channel, one or more radiopaque markers, and a distal tip. One of or more catheters as described herein may have a secondary curve, a primary curve, or no pre-set curves. The primary and secondary curves are not illustrated in the drawings. The distal tip defines the distal most end of each elongate shaft 12. The shaft 12 may, in an alternate embodiment, include an inner channel that is also sized to receive other surgical devices therethrough. For example, the surgical system 10 may be, but is not required to, receive a guidewire such that an over-the-wire technique may be used. That is, a guidewire can be placed through the valve structure into the left ventricle and the distal end of the surgical system 10 or separate steering catheter is inserted over the guidewire into position. In an alternative embodiment, the surgical system 10 or separate steering catheter, or one or more of its shafts 12, may include one or more skive ports that can be used to receive the guidewire therethrough. Such skive ports may be disposed toward or along an outer surface of the shaft 12. In yet another embodiment, the guidewire may not extend through the valve structure into the ventricle. The surgical system, however, may still slide over or along the guidewire, but without the benefit of having the guidewire cross through the valve structure.

In cross-section, a catheter may include an inner liner, a middle reinforcing layer (e.g. a braid), and an outer layer or outer jacket. In addition, the catheter may be a biaxial design that includes an additional outer layer to minimize interaction with the introducer and/or sheath and allow smoother movement of the surgical system. In another embodiment, the catheter would also be able to accommodate different shaped inner catheters to achieve a suitable relationship of the distal catheter tip to the leaflet. For example, this configuration may provide for functionality similar to the use of a 5F/6F 120 mm IM catheter inside an AL type catheter, i.e., a mother and daughter technique. The catheter may be configured to transition in response to operator input to assume different degrees of flexion of the distal tip to account for different patient anatomy.

The longitudinal shape of the catheter can vary as needed. For instance, the catheter can have a shape according to the Amplatz Guide that includes, but is not limited to AL-1, AL-2, AL-3, AL-4, etc. Other common shapes are possible as well. In one example, the catheter may have an outer cross-sectional dimension sized for insertion into the aorta. For instance, the catheter may be either 12 French or 14 French. However, larger, or smaller sized catheters may be used in certain instances. The catheter tip or distal tip may be deflectable or bendable as needed to steer the distal tip into position, for example, when using a steering element as described above. The catheter may also be configured to accommodate different shaped inner catheters.

The catheter has at least one port that extends to the inner channel. As shown, the at least one port could be two or more as needed. The port or ports are spaced a distance from the leading end that is less than a distance between the at least one port and the trailing end. In other words, they are positioned toward the leading end of the catheter. These ports are intended to a) allow for flushing or priming the system prior to introduction to the patient and/or b) allow removal of emboli, such as air and other debris after cutting, and throughout, to provide for hemodynamic monitoring of the blood pressure in the ascending aorta. The ports may also be used for contrast injection, as needed. For instance, when the leaflets get cut, the destruction of the aortic valve may lead to decompensation of coronary output, which is monitored by a local lumen. The system, may, in turn, include a luer fitting on the handle for monitoring and bubble removal. Bubble and debris removal can happen via an active 'vac lock" syringe (pull a vacuum with a syringe and the handle locks in place so holding by the user is not required) on the port for evacuating 50-100ml of blood/air.

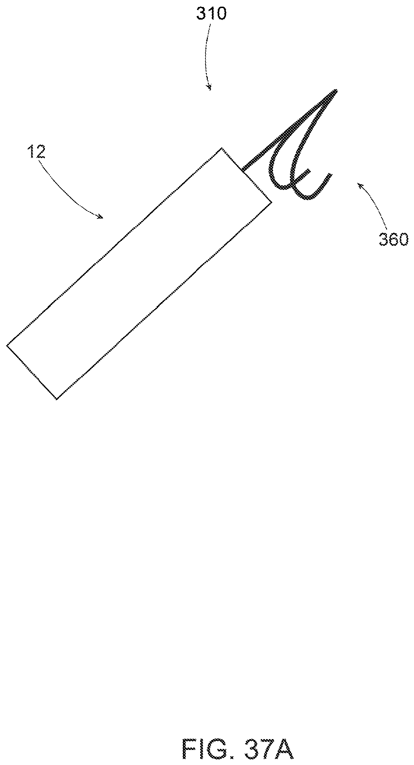

Another embodiment of a surgical system 310 is shown in Figure 37A with a different cutting configuration. Either system 310 or system 10 can be used for mitral valve excision. As shown in Figure 37A, surgical system 310 includes the same features as the surgical system 10 shown in Figures 1-19A and common reference numbers are used to identify features and elements common to both surgical system 10 and surgical system 310. As shown, the surgical system 310 includes an elongate shaft 12 and a cutting element 360 for use in mitral valve excision.

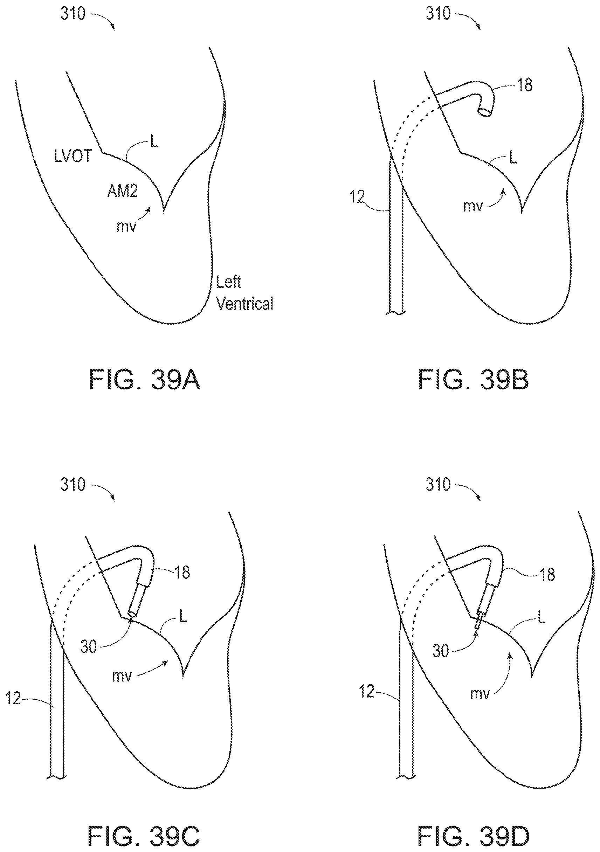

The cutting element 360 includes a first cutter and a second cutter configured as a pair of hooks with a reverse orientation as compared to the cutting hooks shown in Figures 10-19B. More specifically, as shown in Figures 37A, the first cutter may be a first cutting hook with a base portion that extends proximally from a terminal end of a shaft and a curved element that curves outwardly and back toward the terminal end of the shaft. Likewise, the second cutter may be a second hook with a base portion that extends proximally from the terminal end of a shaft and a curved element that curves outwardly and back toward the terminal end of the shaft. In this configuration, distal advancement of the cutting element 360 positions the first and second hooks to lacerate or excise the tissue (native or implanted valve leaflet). Such reverse hook configurations as shown are suitable for either retrograde or antegrade access to the mitral valve, as further shown in Figures 39A-40D.

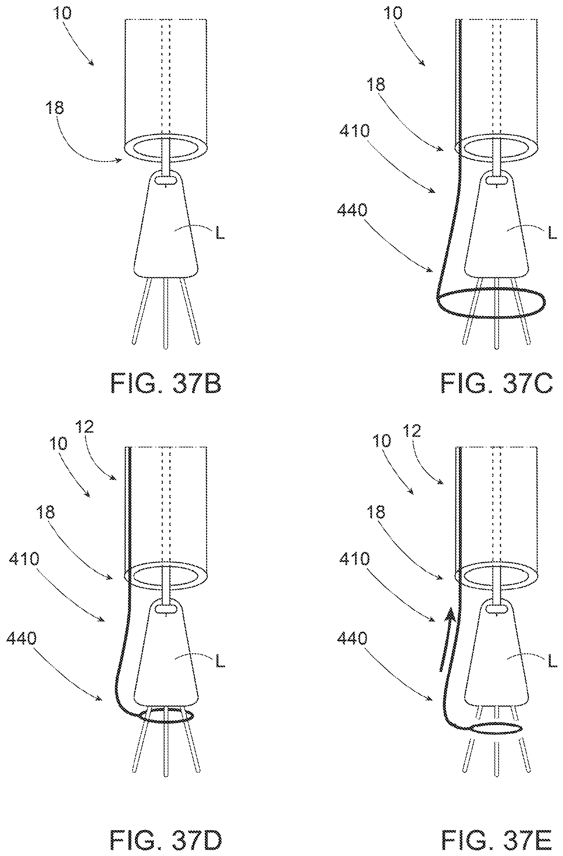

In instances where excision of a mitral valve (native or artificial) is needed, it may be necessary to sever the mitral chordae tendinea. In such a circumstance, the surgical system may include a chordae tendineae cutting assembly 410 as shown in Figures 37B-37E. The cutting assembly 410 may include an elongate shaft 12 and a cutting snare 440. The cutting snare 440 may include an elongate member with a snare that can be used to capture and sever the chordae tendineae. The snare may include an expandable and collapsible loop, which is configured to expand to surround the chordae tendineae and collapsed around the chordae tendineae as needed, as shown in Figures 37C, 37D and 37E. In this example, the snare 440 can be electrified via RF energy to aid in severing the chordae tendineae, as with other cutting elements described in the present disclosure. For instance, the system 410 may include reverse hooks or typical hooks as needed.

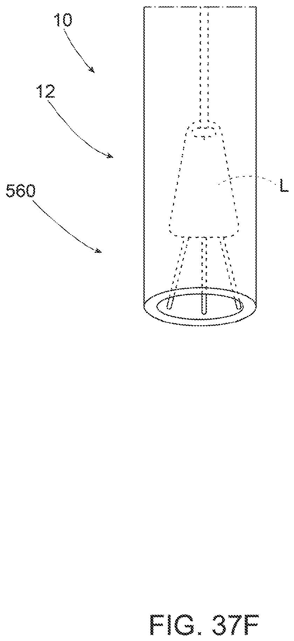

In another embodiment where a mitral valve (native or artificial) excision is needed, the first cutting element 560 can be used to sever the chordae tendineae, as shown Figure 37F. In such an embodiment, the surgical system is configured to fully retract the excised leaflet portion fully or partially into a channel of the elongated shaft via a retraction assembly (not shown). In still another embodiment where a mitral valve (native or artificial) excision is needed, the second cutting element (one or both of the curved hooks) can be used to sever the chordae tendineae.

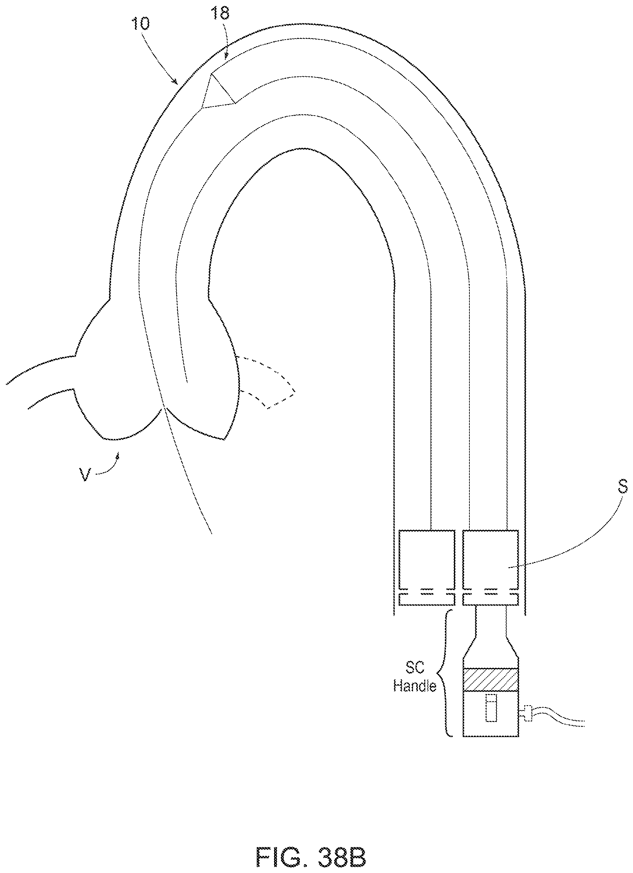

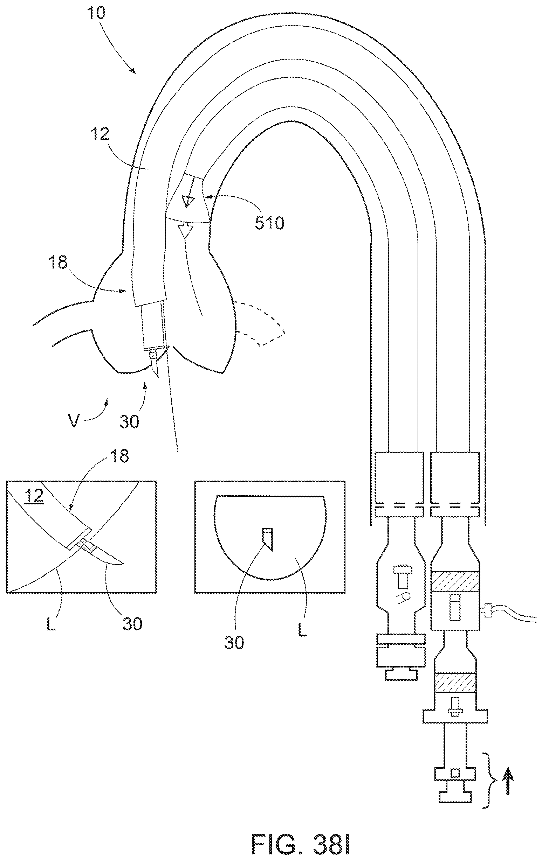

The present disclosure includes various embodiments of extracting a portion of a leaflet of a valve, e.g. a surgical valve, TAVR valve, TMVR valve, native valve, or other valve procedures. Figures 38A-38R illustrate an exemplary method for use of the surgical system 10 as described herein for operating on a valve V. The method may generally include placing a TAVR sheath S in the cardiovascular system. Guidewires AA, LV may be inserted into the TAVR sheath and their distal ends positioned proximate or through the valve V. See Figure 38A. The surgical system or the steerable catheter may be placed over the wire, e.g. the AA or LV. In other embodiments, however, the surgical system or steerable catheter is not placed over the wire. In some embodiments, a hemoshield element 510 may be deployed, as shown in Figures 38D-38F. The method may include, with surgical system 10, advancing a steerable shaft 12 into a cardiovascular system so that its distal end 18 is proximate a valve V, as shown Figures 38B-38C. The catheter may be positioned so that the distal end 18 of the surgical system 10 is in contact with the leaflet of the valve. The piercing element 30 may be advanced in a distal direction to form a pierced opening in the leaflet L of the valve V, as shown in Figure 38I. In one example, but not required, forming the pierced opening with the piercing element 30 comprises supplying electrical energy to piercing element when in contact with the leaflet.

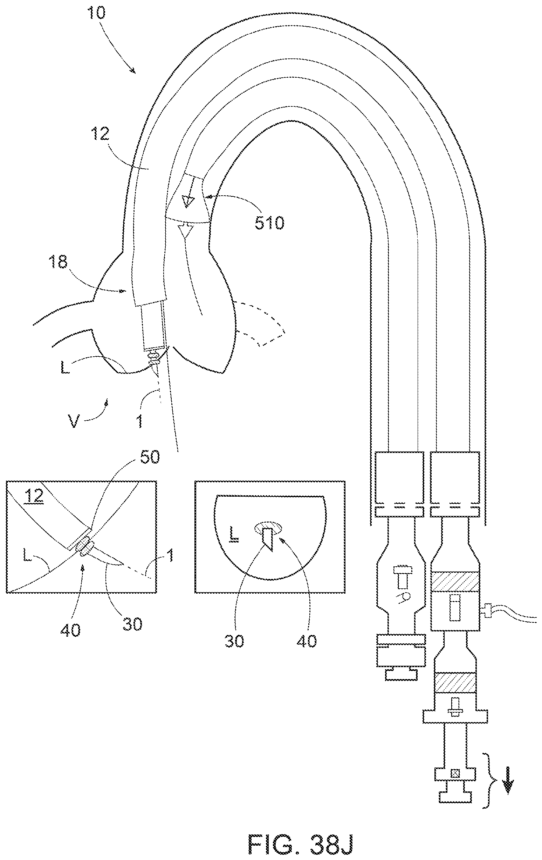

As shown in Figure 38I, the user may advance a capture element 40 along the central axis and through the pierced opening in the leaflet L. As depicted in Figure 38I, the piercing element and capture element may reside on the same assembly. As shown in Figure 38J, advancing the capture element 40 through the pierced opening in the leaflet L will further include deploying the capture element 30 through the pierced opening in an insertion configuration where a first expandable portion and a second expandable portion of the capture element 30 are collapsed toward a central axis 1. Then, the user can transition the capture element 30 from an insertion configuration into a capturing configuration, where the capture element seizes the leaflet L, as shown in Figure 38J. Seizing the leaflet L with the capture element 30 may further include causing the capture element 30 to transition from the insertion configuration into an expanded configuration where the first expandable portion and the second expandable portion are expanded outwardly away from the central axis 1. Furthermore, the user can cause the capture element 30 to transition from the expanded configuration into a captured configuration where the first expandable portion and the second expandable portion to seize the leaflet L.

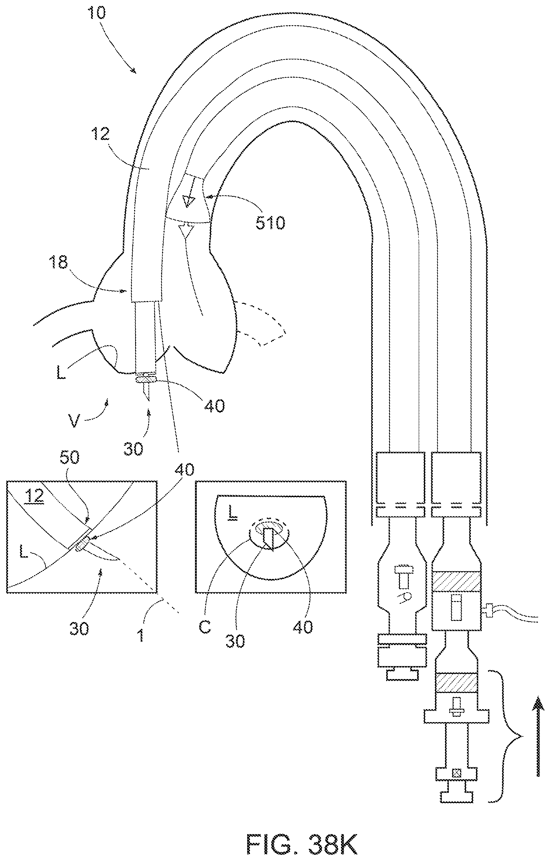

As shown in Figure 38K, with the leaflet L seized by the capture element, the surgical system 10 can form a curved opening C in the leaflet proximate the pierced opening with a first cutting element 50. Forming the curved opening C includes advancing the first cutting element 50 into contact with the leaflet L proximate the pierced opening. In one example, forming the curve opening C includes supplying electrical energy to the first cutting element when in contact with the leaflet. In these examples, the curved opening C extends around a portion of the central axis. For instance, the curved cut is substantially C-shaped and/or extends around a majority of the central axis.

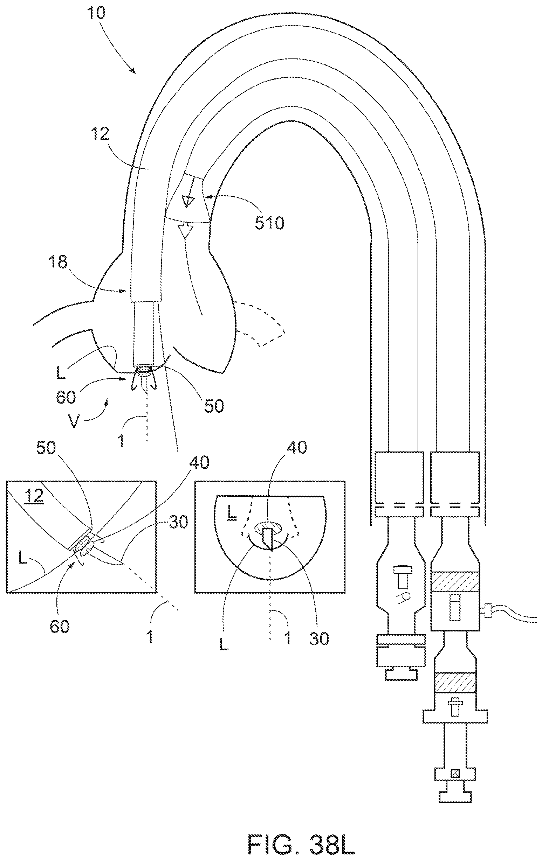

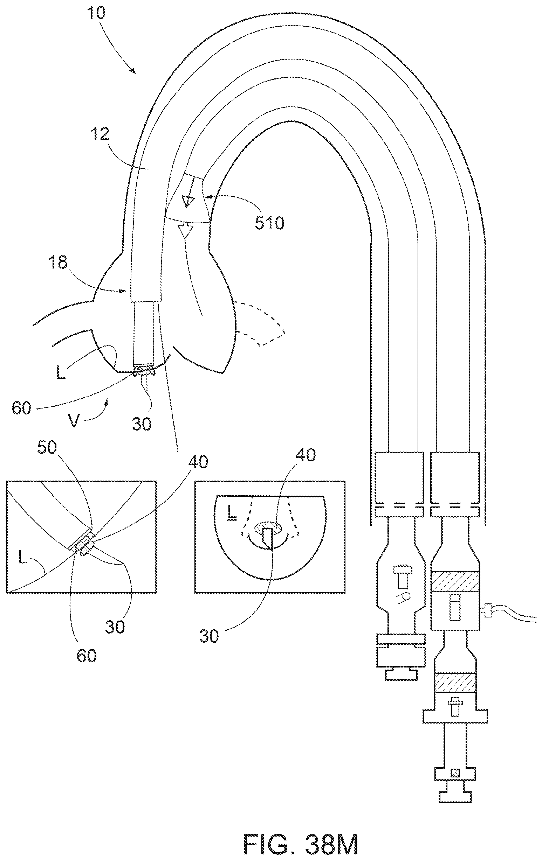

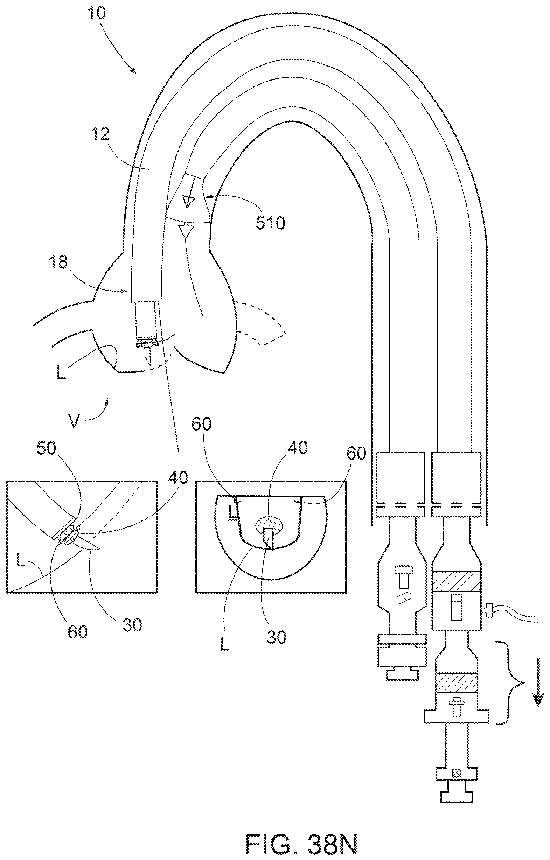

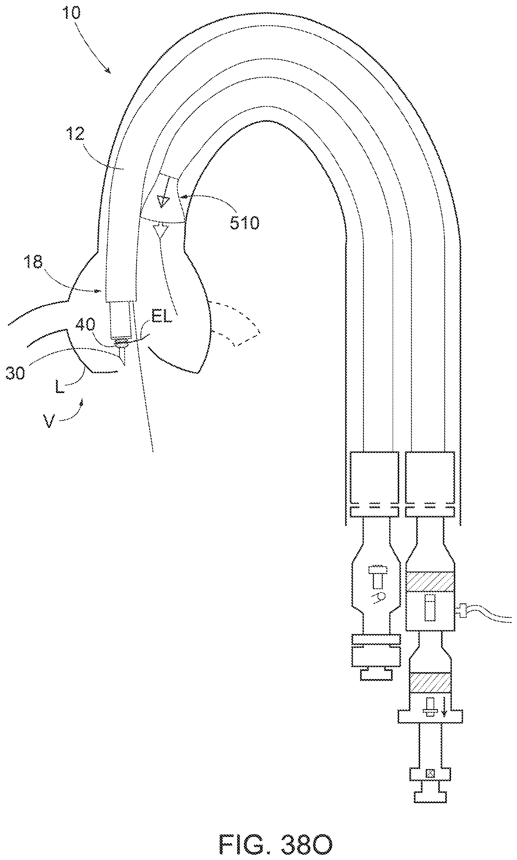

As shown in Figure 38L, while seizing the leaflet L with the capture element 40 to maintain the relative position of the leaflet relative to the shaft, a second cutting element 60 is inserted through the leaflet L. The second cutting element 60 has first and second cutters, as described above. More specifically, the first cutter and the second cutter are advanced into contact with the leaflet L through the curved opening. Then, as shown in the transition of Figure 38M to Figure 38N, the first cutter and the second cutter may splay apart while supplying electrical energy to the first and second cutters. As shown in Figures 38N and 38O, the first and second cutters are also retracted to excise the leaflet L, thereby forming an excised portion EL of the leaflet L. Furthermore, during the splaying, the user retracts the capture element 40, while seizing the cut leaflet portion EL, toward the proximal end of the steerable shaft 12.

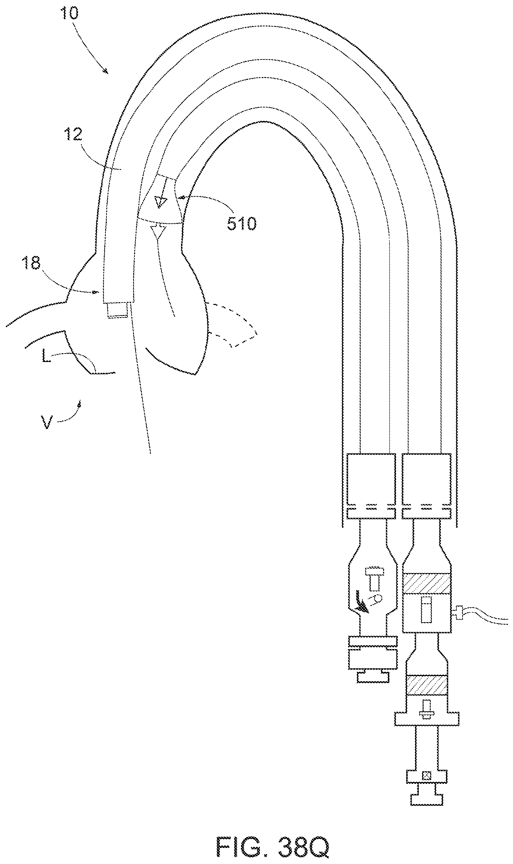

While the first and second cutters are in the cutting configuration and moving in the proximal direction, the capture element 40 maintains its relative position while still grabbing the leaflet L. By placing some distally directed force on the capture element 40, tension is maintained in the leaflet L, allowed the first and second cutters 60 to splay apart, either through shape memory, or by following the natural anatomy of the valve V. The first and second cutters separate and splay while moving in the proximal direction, excising a portion of the leaflet L (excised portion EL shown in Fig, 38O). By holding or advancing the leaflet L in place while splaying the first and second cutters, the amount of leaflet L that can be removed from the valve may be optimized. In other words, holding the leaflet L while splaying the cutters to excise the leaflet can maximize the amount of tissue that can be removed from a particular leaflet. In this regard, the excised leaflet EL remains attached to the capturing element 40 for subsequent removal from the patient as depicted in Figures 38O through 38R. Figure 38Q also shows regurgitation adjustment of the hemoshield in response to leaflet excision.

The present disclosure described embodiments for excision portions of a leaflet in an aortic valve. However, the embodiments of the disclosure are also suitable for excision portions of a mitral valve. Figures 39A-40D illustrate a procedure for excising mitral valves MV. A surgical system 310 may be used to excise mitral valve portions. More specifically, the surgical system 310 may include the same elements and features of the surgical system 10 and common reference numbers are used to denote features common to each surgical system 10 and 310. Furthermore, many of the same functions used for aortic valves, e.g., piercing via piercing element, capturing the leaflet via capture element, forming a curved opening with the first cutting element 50, excising the leaflet with the second cutting element, are used for excising mitral valves portions. However, different features will use different reference numbers. Turning to Figures 39A-39D, a procedure using system 310 for antegrade access to, and removal of, portions of a mitral valve is shown. More specifically, as shown in Figure 39B, a steerable shaft 12 is advanced into a cardiovascular system so that its distal end 18 enters a right atrium of a heart and is positioned proximate a superior side of a mitral valve MV. Then, as shown in Figure 39C, the surgical system is advanced through the steerable catheter and into contact with a mitral valve leaflet. Then, as shown in Figure 39D, the method includes advancing a piercing element 30 into contact with a leaflet L of the mitral valve MV. Next, the method includes piercing, with the piercing element 30, the leaflet L of the mitral valve to form a pierced opening in the leaflet of the mitral valve MV. A capture element 30 is then advanced through the pierced opening in the leaflet and transitioned into a deployed configuration, i.e. the capture element is not yet expanded. The method then includes transitioning the capture element 40 from the deployed configuration into a capturing configuration to seize the leaflet L with the capture element 40. With the leaflet seized by the capture element 30, the method includes forming a curved opening in the leaflet L proximate the pierced opening with a first cutting element. While seizing the leaflet L with the capture element 40 to maintain the relative position of the leaflet relative to the shaft, the method includes inserting a second cutting element 60 having a first and second cutters through the leaflet. Then, the user can move the second cutting element 60 in a proximal direction, while splaying apart the first cutter and a second cutter away from each other, to remove a portion of the leaflet from the mitral valve (not shown). Here the first and second cutters can be cutter hooks or reverse cutting hooks, and movement may be either pushing or pulling the cutting element to severe the leaflet portion.

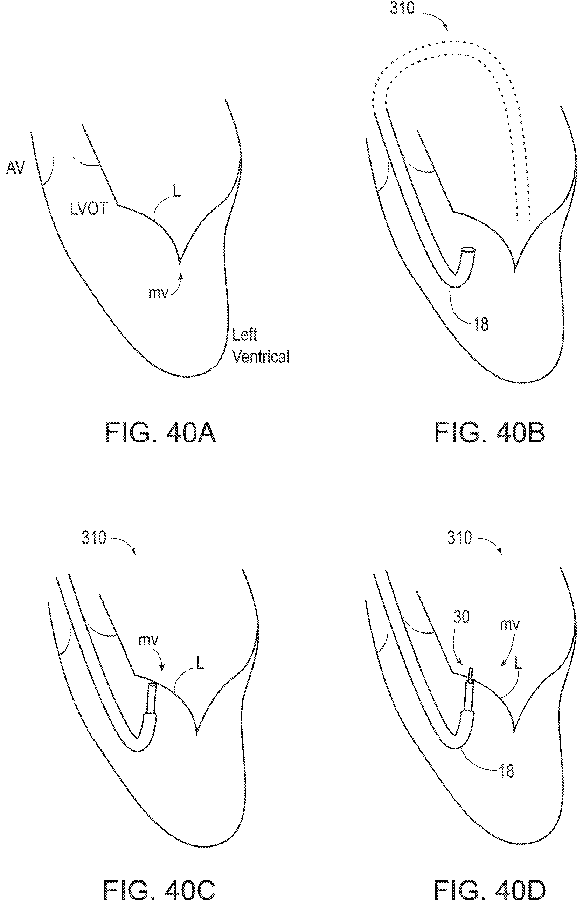

Turning to Figures 40A-40D, a procedure using system 310 for retrograde access to, and removal of, the mitral valve. More specifically, as shown in Figure 40B, the method includes advancing a steerable shaft 12 into a cardiovascular system so that its distal end 18 is positioned proximate an inferior side of a mitral valve. Then, as shown in Figure 40C, the surgical system is advanced through the steerable catheter and into contact with a mitral valve leaflet. Then, the method includes advancing a piercing element 30 into contact with the inferior side leaflet L of the mitral valve MV. Next, the method includes piercing, with the piercing element 30, the leaflet L of the mitral valve to form a pierced opening in the leaflet of the mitral valve. A capture element 40 is then advanced through the pierced opening in the leaflet and transitioned into a deployed configuration (not shown). The method then includes transitioning the capture element 40 from the deployed configuration into a capturing configuration, where the capture element is expanded, to seize the leaflet with the capture element 40. With the leaflet L seized by the capture element 40, the method includes forming a curved cut in the leaflet proximate the pierced opening with a first cutting element 50. While seizing the leaflet with the capture element 40 to maintain the relative position of the leaflet L relative to the shaft, the method includes inserting a second cutting element having a first and second cutters through the leaflet. Then, the user can move the second cutting element 60 proximally, while splaying apart the first cutter and a second cutter away from each other, to remove a portion of the leaflet from the mitral valve. Here the first and second cutters can be cutter hooks or reverse cutting hooks, and movement may be either pushing or pulling the cutting element to severe the leaflet portion.

In either case, antegrade (Figures 39A-39D) or retrograde access (Figures 40A-40D), the method may include severing mitral chordae tendineae with a cutting element. In one example, severing mitral chordae tendineae with a cutting element includes capturing the chordae tendineae with a snare 440 and causing the snare 440 to sever the chordae tendineae, as described above. In another example, severing mitral chordae tendineae with a cutting element includes retracting the seized portion of the leaflet into a channel of the elongated shaft and using the cutting edge of the first cutting element 560 to sever the chordae tendineae. In still another embodiment, one or both curved hooks of the second cutting element may be used to sever the chordae tendineae.

In all cases, the surgical system may be used with a native valve, an implanted artificial valve, or a valve that is implanted on a surgical ring, or a valve-in-valve.

It will be appreciated by those skilled in the art that various modifications and alterations of the present disclosure can be made without departing from the broad scope of the appended claims. Some of these have been discussed above and others will be apparent to those skilled in the art. The scope of the present disclosure is limited only by the claims.
The following numbered paragraphs define particular embodiments of the present invention:
1. A surgical system, comprising:
   a shaft having a proximal end and a distal end spaced from the proximal end along a central axis;
   a capture element carried by the shaft, the capture element having a first expandable portion and a second expandable portion spaced relative to the first expandable portion along the central axis, the first expandable portion and the second expandable portion being configured to expand in order to seize a portion of a leaflet of the valve.
2. A surgical system. comprising:
   a shaft having a proximal end, a distal end spaced from the proximal end along a central axis, and an outer perimeter that extends around the central axis;
   a cutting element carried by the shaft, the cutting element having a curved cutting edge that extends around a portion of the outer perimeter of the distal end of the shaft, the curved cutting edge configured to create a curved opening in a leaflet of a valve.
3. A surgical system, comprising:
   a shaft having a proximal end and a distal end spaced from the proximal end along a central axis;
   a piercing element configured to puncture a leaflet of a valve; and
   a capture element having a first expandable portion and a second expandable portion spaced relative to the first expandable portion along the central axis, the first expandable portion and the second expandable portion being configured to expand in order to seize a portion of a leaflet of the valve.
4. A surgical system, comprising:
   a shaft having a proximal end, a distal end spaced from the proximal end along a central axis, and an outer perimeter the extends around the central axis;
   a piercing element configured to puncture a leaflet of a valve; and
   a cutting element carried by the shaft and moveable relative to the piercing element, the cutting element having a curved cutting edge that extends around a portion of the outer perimeter of the distal end of the shaft, the curved cutting edge configured to create a curved opening in a leaflet of a valve.
5. A surgical system, comprising:
   a shaft having a proximal end and a distal end spaced from the proximal end along a central axis;
   a piercing element configured to puncture a leaflet of a valve; and
   a cutting element having a first cutter and a second cutter carried at least partly by the shaft, the first cutter and the second cutter being configured to separate outward away from each other while moving in a proximal direction to cut away a portion of the leaflet while the capture element seizes the leaflet of the valve.
6. A surgical system, comprising:
   a shaft having a proximal end, a distal end spaced from the proximal end along a central axis, and an outer perimeter the extends around the central axis;
   a capture element having a first expandable portion and a second expandable portion spaced relative to the first expandable portion along the central axis, the first expandable portion and the second expandable portion being configured to expand in order to seize a portion of a leaflet of the valve; and
   a cutting element moveable relative to the shaft, the cutting element having a curved cutting edge that extends around a portion of the outer perimeter of the distal end of the shaft, the curved cutting edge configured to create a curved opening in a leaflet of a valve.
7. A surgical system, comprising:
   a shaft having a proximal end and a distal end spaced from the proximal end along a central axis;
   a capture element having a first expandable portion and a second expandable portion spaced relative to the first expandable portion along the central axis, the first expandable portion and the second expandable portion being configured to expand in order to seize a portion of a leaflet of the valve; and
   a cutting element having a first cutter and a second cutter carried at least partly by the shaft, the first cutter and the second cutter being configured to separate outward away from each other while moving in a proximal direction to cut away a portion of the leaflet while the capture element seizes the leaflet of the valve.
8. A surgical system, comprising:
   a shaft having a proximal end and a distal end spaced from the proximal end along a central axis;
   a first cutting element carried by the shaft, the cutting element having a curved cutting edge that extends around a portion of the outer perimeter of the distal end of the shaft, the curved cutting edge configured to create a curved opening in a leaflet of a valve; and
   a second cutting element having a first cutter and a second cutter carried at least partly by the shaft, the first cutter and the second cutter being configured to separate outward away from each other while moving in a proximal direction to cut away a portion of the leaflet while the capture element seizes the leaflet of the valve.
9. A surgical system, comprising:
   a shaft having a proximal end and a distal end spaced from the proximal end along a central axis;
   a piercing element configured to puncture a leaflet of a valve;
   a capture element having a first expandable portion and a second expandable portion spaced relative to the first expandable portion along the central axis, the first expandable portion and the second expandable portion being configured to expand in order to seize a portion of a leaflet of the valve;
   a first cutting element carried by the shaft, the cutting element having a curved cutting edge that extends around a portion of the outer perimeter of the distal end of the shaft, the curved cutting edge configured to create a curved opening in a leaflet of a valve; and
   a second cutting element having a first cutter and a second cutter carried at least partly by the shaft, the first cutter and the second cutter being configured to separate outward away from each other while moving in a proximal direction to cut away a portion of the leaflet while the capture element seizes the leaflet of the valve.
10. A surgical system, comprising:
   a shaft having a proximal end and a distal end spaced from the proximal end along a central axis;
   a capture element carried by the shaft, the capture element having an expandable portion and fixed collar spaced relative to the expandable portion along the central axis, wherein the expandable portion is configured to expand in order to seize a portion of a leaflet of the valve with the collar.
11. The surgical system of according to any one of paragraphs 1 to 10, wherein the shaft includes an inner channel that extends from the distal end toward the proximal end.
12. The surgical system according to any one of paragraphs 1 to 11, further comprising a steering element configured to guide the distal end of the shaft toward a target location of a valve.
13. The surgical system according to paragraphs 2, 4, 6, 8, and 9 through 12, wherein the curved cutting edge of the cutting element has a C-shape.
14. The surgical system according to paragraphs 2, 4, 6, 8, and 9 through 13, wherein the curved cutting edge of the cutting element is further angled along a plane that intersects the central axis.
15. The surgical system according to paragraphs 2, 4, 6, 8, and 9 through 14, wherein the curved cutting edge of the cutting element is a sharp element.
16. The surgical system according to paragraphs 2, 4, 6, 8, and 9 through 15, wherein the curved cutting edge is an electrode.
17. The surgical system of any one of paragraphs 3, 4, 5, and 9 through 16, wherein the shaft has an inner channel, wherein the piercing element is movable between a retracted configuration where a distal tip of piercing element is located in the inner channel, and a deployed configuration where the distal tip of the piercing element is located outside of the inner channel.
18. The surgical system of any one of paragraphs 3, 4, 5, 9 through 12, and 17, wherein a distal tip of piercing element is sharp tip.
19. The surgical system of any one of paragraphs 3, 4, 5, 9 through 12, and 17, wherein a distal tip of piercing element is an electrode.
20. The surgical system of any one of paragraphs 3, 4, 5, 9 through 12, and 17, wherein a distal tip of piercing element has a circular cross-section with sharp leading edge.
21. The surgical system according to any one of paragraphs 1, 3, 6, 7, and 9 through 12, wherein the capture element includes:
   a) an insertion configuration where the first expandable portion and the second expandable portion are collapsed toward the central axis,
   b) an expanded configuration where the first expandable portion and the second expandable portion are expanded outwardly away from the central axis, and
   c) a captured configuration where at least a portion the first expandable portion and at least a portion of the second expandable portion are positioned to seize a portion of the leaflet while in the expanded configuration.
22. The surgical system according to any one of paragraphs 1, 3, 6, 7, 9 through 12, and 21, wherein the capture element further includes an expandable braid and a collar disposed along the expandable braid and configured to separate the expandable braid into the first expandable portion and the second expandable portion.
23. The surgical system according to any one of paragraphs 1, 3, 6, 7, 9 through 12, and 20 through 22, wherein the capture element further includes a fixed collar and an expandable portion positioned distally with respect to the fixed collar.
24. The surgical system of paragraph 23, wherein the expandable braid is a nitinol braid.
25. The surgical system according to paragraphs 1, 3, 6, 7, 9 through 12, and 24, wherein the first expandable portion and the second expandable portion are first and second wire portions, respectively, that extends around a shaft, wherein the first and second wire portions are configured to expand radially outward.
26. The surgical system according to paragraphs 1, 3, 6, 7, 9 through 12, and 25, wherein the first expandable portion and the second expandable portion are first and second sets of longitudinal wires respectively, wherein the first and second set of longitudinal wires configured to fold outwardly.
27. The surgical system according to any one of paragraphs 1, 3, 6, 7, 9 through 12, and 21, wherein the first expandable portion and the second expandable portion is a first balloon and a second balloon, respectively, and a collar is located between the first balloon and the second balloon.
28. The surgical system according to any one of paragraphs 3, 4, 5, 9 through 12 and 17 through 20, further comprising at least one actuator configured to move the piercing element along the shaft.
29. The surgical system according to any one of paragraphs 1, 3, 6, 7, and 9 through 12, and 19 through 27, further comprising at least one actuator configured to actuate the capture element to seize a portion of the leaflet.
30. The surgical system according to any one of paragraphs 5, 7, 8, and 9 through 12, wherein the shaft has an inner channel, wherein the first cutter and the second cutter have
   a) an insertion configuration, where terminal ends of the first cutter and the second cutter are located inside the inner channel,
   b) a deployed configuration, where terminal ends of the first cutter and the second cutter is located outside of the inner channel, and
   c) a cutting configuration, where the terminal ends of the first cutter and the second cutter are splayed apart with respect to each other.
31. The surgical system according to paragraph 30, wherein the cutting element has at least one actuator configured to cause the first cutter and the second cutter to transition through the insertion configuration, the deployed configuration, and the cutting configuration.
32. The surgical system according to any one of paragraphs 5, 7, 8, 9 through 12, 30, and 31, wherein the first cutter and the second cutter each include a) an elongate shaft and b) a cutting hook that extends from the elongate shaft.
33. The surgical system of paragraph 32, wherein the cutting hook has a curved body and at least one cutting edge.
34. The surgical system of according to paragraphs 8 and 9 through 11, 30, and 31, wherein in a fist mode of operation, the first cutting element and the second cutting element are configured to advance in tandem, and in a second mode of operation, the second cutting element is configured to advance relative to the first cutting element and the shaft.
35. The surgical system according to any one of paragraphs 3, 4, 5, 9 through 34, further comprising an electrode coupled to the piercing element that is responsive to electrical energy.
36. The surgical system according to any one of paragraphs 2, 4, 6, 8, and 9 through 35, further comprising an electrode coupled to the cutting element that is responsive to electrical energy.
37. The surgical system according to any one of paragraphs 5, 7, 8, and 9 through 36, further comprising an electrode coupled to the cutting element that is responsive to electrical energy.
38. The surgical system according to any one of paragraphs 35 through 37, further comprising an electrosurgical unit configured to supply electric energy to the electrode.
39. The surgical system according to any one of paragraphs 1, 3, 6, 7, and 9 through 12, and 19 through 38, wherein the first expandable portion and the second expandable portion of the capture element are configured to expand substantially simultaneously.
40. The surgical system according to any one of paragraphs 1, 3, 6, 7, and 9 through 12, and 19 through 38, wherein the first expandable portion and the second expandable portion of the capturing element are configured to expand sequentially.
41. The surgical system according to any one of paragraphs 1 to 38, wherein the shaft is a first shaft, and further comprising a second shaft separate from the first shaft.
42. The surgical system according to paragraph 41, wherein the second shaft carries at least one cutting element.
43. The surgical system according to paragraph 41, wherein the second shaft is configured to deliver a non-ionic solution.
44. The surgical system according to any one of paragraphs 1 to 43, further comprising a positioning element earned by the shaft, wherein the positioning element is sized and shaped to stabilize the position of a distal end of the shaft relative to tissue.
45. The surgical system according to paragraph 44, wherein the positioning element is a slidable tube that extends around the distal end of the shaft.
46. The surgical system according to paragraph 44, wherein the positioning element is a hood carried by the distal end of the shaft.
47. The surgical system according to paragraph 44, wherein the positioning element is an adjustable loop carried by the distal end of the shaft.
48. A method, comprising:
   advancing a steerable shaft into a cardiovascular system so that its distal end is proximate a valve, wherein the steerable shaft has a central axis;
   advancing a piercing element along the central axis and into contact with a leaflet of the valve;
   piercing, with the piercing element, the leaflet of the valve to form a pierced opening in the leaflet of the valve;
   advancing a capture element along the central axis and through the pierced opening in the leaflet;
   deploying the capture element from an insertion configuration into a capturing configuration;
   transitioning the capture element from the deployed configuration into a capturing configuration to seize the leaflet with the capture element;
   with the leaflet seized by the capture element, forming a curved cut in the leaflet proximate the pierced opening with a first cutting element, wherein the curved cut extends around a portion of the central axis; and
   while seizing the leaflet with the capture element to maintain the relative position of the leaflet relative to the shaft, inserting a second cutting element having a first and second cutters through the leaflet;
   retracting the second cutting element, while splaying apart the first cutter and a second cutter away from each other, to remove a portion of the leaflet from the valve.
49. The method of paragraph 48, further comprising, before advancing the steerable catheter in the cardiovascular system, placing a sheath in the cardiovascular system.
50. The method of paragraph 48, wherein advancing the capture element through the pierced opening in the leaflet, further comprises deploying the capture element through the pierced opening in an insertion configuration where a first expandable portion and a second expandable portion of the capture element are collapsed toward a central axis.
51. The method of paragraph 48, wherein seizing the leaflet with the capture element further comprises:
   causing the capture element to transition from the insertion configuration into an expanded configuration where the first expandable portion and the second expandable portion are expanded outwardly away from the central axis; and
   then, causing the capture element to transition from the expanded configuration into a captured configuration where the first expandable portion and the second expandable portion to seize the leaflet.
52. The method of paragraph 48, wherein forming the curved cut in the leaflet proximate the pierced opening further comprises advancing the first cutting element into contact with the leaflet proximate the pierced opening.
53. The method of paragraph 48, wherein the curved cut is substantially C-shaped.
54. The method of paragraph 48, wherein the curved cut extends around a majority of the central axis.
55. The method of paragraph 48, wherein piercing, with the piercing element, to form the pierced opening in the leaflet of the valve, further comprises supplying electrical energy to piercing element when in contact with the leaflet.
56. The method of paragraph 48, wherein forming a curve cut in the leaflet further comprises supplying electrical energy to the first cutting element when in contact with the leaflet.
57. The method of paragraph 48, further comprising retracting the capture element with the cut leaflet portion toward the proximal end of the steerable shaft.
58. The method of paragraph 48, further comprising retracting the capture element with the cut leaflet portion into the proximal end of the steerable shaft.
59. The method of paragraph 48, wherein cutting the leaflet with the first cutter and the second cutter further comprises:
   advancing the first cutter and the second cutter into contact with the leaflet;
   splaying the first cutter and the second cutter apart while supplying electrical energy to the first and second cutters; and
   retracting the first cutter and the second cutter to excise the leaflet, thereby forming an excised portion of the leaflet.
60. The method according to paragraph 48, wherein retracting the first cutter and the second cutter to excise the leaflet includes applying distally directed force on the capture element to apply tension on the leaflet, thereby allowing the first and second cutters to splay apart.
61. The surgical system according to any one of paragraphs 5, 7, 8 and 9, wherein the first and second cutter have first and second reverse cutting hooks, respectively.
62. The surgical system according to paragraph 61, wherein the first and second cutting hooks each have a base portion that extends proximally from a terminal end of a shaft and a curved element that curves outwardly and back toward the terminal end of the shaft.
63. The surgical system according to any one of paragraphs 61 to 62, further comprising an electrode coupled to the piercing element that is responsive to electrical energy.
64. The surgical system according to any one of paragraphs 5 through 47, further comprising a cutting snare.
65. The surgical system according to paragraph 64, wherein the cutting snare may include an elongate member with a snare configured to extend from a retracted configuration into a snaring configuration around a target tissue.
66. The surgical system according to either paragraph 64 or 65, wherein the cutting snare includes an expandable and collapsible loop that is configured to expand to surround the tissue and collapsed around the tissue.
67. The surgical system according to any one of paragraphs 64 through 66, further comprising an electrode coupled to the cutting snare that is responsive to electrical energy.
68. The surgical system according to any one of paragraphs 2, 4, 6, 8, 9 and 13 through 16, wherein the curved cutting edge is also configured to sever chordae tendineae.
69. The surgical system according to any one of paragraphs 61 through 68, wherein the elongated shaft includes a retracting assembly configured to retract a severed portion of the leaflet in a channel of the elongated shaft to facilitate severing the chordae tendineae with the curved cutting edge.
70. The surgical system according to according to any one of paragraphs 61 through 69, wherein the elongated shaft includes a retracting assembly configured to retract a severed portion of the leaflet in a channel of the elongated shaft to facilitate severing the chordae tendineae with the cutting hooks.
71. A method, comprising:
   advancing a steerable shaft into a cardiovascular system so that its distal end enters a right atrium of a heart and is positioned proximate a superior side of a mitral valve;
   advancing a piercing element into contact with a leaflet of the mitral valve;
   piercing, with the piercing element, the leaflet of the mitral valve to form a pierced opening in the leaflet of the mitral valve;
   advancing a capture element through the pierced opening in the leaflet;
   deploying the capture element from an insertion configuration into a deployed configuration;
   transitioning the capture element from the deployed configuration into a capturing configuration to seize the leaflet with the capture element;
   with the leaflet seized by the capture element, forming a curved opening in the leaflet proximate the pierced opening with a first cutting element, wherein the curved opening extends around a portion of an axis of the elongated shaft; and
   while seizing the leaflet with the capture element to maintain the relative position of the leaflet relative to the shaft, inserting a second cutting element having a first and second cutters through the leaflet;
   moving the second cutting element, while splaying apart the first cutter and a second cutter away from each other, to remove a portion of the leaflet from the mitral valve.
72. A method, comprising:
   advancing a steerable shaft into a cardiovascular system so that its distal end enters an aortic valve and is positioned proximate an inferior side of a leaflet of a mitral valve;
   advancing a piercing element into contact with the inferior side of a leaflet of the mitral valve;
   piercing, with the piercing element, the leaflet of the mitral valve to form a pierced opening in the leaflet of the mitral valve;
   advancing a capture element through the pierced opening in the leaflet;
   deploying the capture element from an insertion configuration into a deployed configuration;
   transitioning the capture element from the deployed configuration into a capturing configuration to seize the leaflet with the capture element;
   with the leaflet seized by the capture element, forming a curved opening in the leaflet proximate the pierced opening with a first cutting element, wherein the curved opening extends around a portion of an axis of the elongated shaft; and
   while seizing the leaflet with the capture element to maintain the relative position of the leaflet relative to the shaft, inserting a second cutting element having a first and second cutters through the leaflet;
   moving the second cutting element, while splaying apart the first cutter and a second cutter away from each other, to remove a portion of the leaflet from the mitral valve.
73. The method of paragraph 71 or paragraph 72, wherein moving the second cutting element includes pushing the first and second cutters in a distal direction to remove the portion of the leaflet from the mitral valve.
74. The method of paragraph 71 or paragraph 72, wherein moving the second cutting element includes pulling the first and second cutters in a proximal direction to remove the portion of the leaflet from the mitral valve.
75. The method of any one of paragraphs 71, 72, and 73, wherein advancing a steerable shaft into the cardiovascular system is antegrade.
76. The method of any one of paragraphs 71 through 75, wherein advancing a steerable shaft into the cardiovascular system is retrograde.
77. The method of any one of paragraphs 71 through 76, further comprising, before advancing the steerable catheter in the cardiovascular system, placing a sheath in the cardiovascular system.
78. The method of any one of paragraphs 71 through 77, wherein advancing the capture element through the pierced opening in the leaflet, further comprises deploying the capture element through the pierced opening in an insertion configuration where a first expandable portion and a second expandable portion of the capture element are collapsed toward a central axis.
79. The method of any one of paragraphs 71 through 78, wherein seizing the leaflet with the capture element further comprises:
   causing the capture element to transition from the insertion configuration into an expanded configuration where the first expandable portion and the second expandable portion are expanded outwardly away from the central axis; and
   then, causing the capture element to transition from the expanded configuration into a captured configuration where the first expandable portion and the second expandable portion to seize the leaflet.
80. The method of any one of paragraphs 71 through 79, wherein forming the curved opening in the leaflet proximate the pierced opening further comprises advancing the first cutting element into contact with the leaflet proximate the pierced opening.
81. The method of any one of paragraphs 71 through 79, wherein the curved opening is substantially C-shaped.
82. The method of any one of paragraphs 71-81, wherein the curved opening extends around a portion of the axis of the elongated shaft.
83. The method according to any one of paragraphs 71 through 82, wherein piercing, with the piercing element, to form the pierced opening in the leaflet of the valve, further comprises supplying electrical energy to piercing element when in contact with the leaflet.
84. The method according to any one of paragraphs 71 through 83, wherein forming a curve cut in the leaflet further comprises supplying electrical energy to the first cutting element when in contact with the leaflet.
85. The method according to any one of paragraphs 71 through 84, further comprising retracting the capture element with the cut leaflet portion toward the proximal end of the steerable shaft.
86. The method according to any one of paragraphs 71 through 84, wherein cutting the leaflet with the first cutter and the second cutter further comprises:
   advancing the first cutter and the second cutter into contact with the leaflet;
   splaying the first cutter and the second cutter apart while supplying electrical energy to the first and second cutters; and
   retracting the first cutter and the second cutter to excise the leaflet, thereby forming an excised portion of the leaflet.
87. A method, comprising:
   advancing a steerable shaft into a cardiovascular system so that its distal end is positioned proximate a leaflet of a mitral valve;
   piercing, with a piercing element, the leaflet of the mitral valve to form a pierced opening;
   advancing a capture element through the pierced opening in the leaflet;
   seizing the leaflet with the capture element;
   with the leaflet seized by the capture element, forming a curved opening in the leaflet proximate the pierced opening; and
   while seizing the leaflet with the capture element to maintain the relative position of the leaflet relative to the shaft, inserting a cutting element having a first and second cutters through the leaflet;
   moving the cutting element to remove a portion of the leaflet from the mitral valve; and
   severing mitral chordae tendineae with a cutting element.
88. The method of paragraph 87, wherein severing mitral chordae tendineae with a cutting element includes:
   capturing the chordae tendineae with a snare; and
   causing the snare to sever the chordae tendineae.
89. The method of paragraph 87 or paragraph 88, wherein severing mitral chordae tendineae with a cutting element includes retracting the seized portion of the leaflet into a channel of the elongated shaft and causing a curved cutting edge to the sever the chordae tendineae.
90. The method according to any one of paragraphs 87 through 89, wherein the valve is a native valve.
91. The method according to any one of paragraphs 87 through 89, wherein the valve is an artificial valve.
92. The method according to any one of paragraphs 87 through 89, wherein the valve is implanted in a surgical ring.
93. A method, comprising:
   advancing a steerable shaft into a cardiovascular system so that its distal end is proximate a valve, wherein the steerable shaft has a central axis;
   advancing a piercing element along the central axis and into contact with a leaflet of the valve;
   piercing, with the piercing element, the leaflet of the valve to form a pierced opening in the leaflet of the valve;
   advancing a capture element along the central axis and through the pierced opening in the leaflet;
   deploying the capture element from an insertion configuration into a capturing configuration to seize the leaflet.
94. A method, comprising:
   capturing a portion of a leaflet of a valve with a capture element to seize the leaflet;
   with the leaflet seized by the capture element, forming a curved opening in the leaflet proximate the pierced opening with a first cutting element, wherein the curved cut extends around a portion of the central axis; and
   while seizing the leaflet with the capture element to maintain the relative position of the leaflet relative to the shaft, inserting a second cutting element having a first and second cutters through the leaflet;
   retracting the second cutting element, while splaying apart the first cutter and a second cutter away from each other, to remove a portion of the leaflet from the valve.
95. A method, comprising:
   advancing a steerable shaft into a cardiovascular system so that its distal end enters a right atrium of a heart and is positioned proximate a superior side of a mitral valve;
   advancing a piercing element into contact with a leaflet of the mitral valve;
   piercing, with the piercing element, the leaflet of the mitral valve to form a pierced opening in the leaflet of the mitral valve; and
   advancing a capture element through the pierced opening in the leaflet to seize the leaflet.
96. A method, comprising:
   advancing a steerable shaft into a cardiovascular system so that its distal end enters a right atrium of a heart and is positioned proximate a superior side of a mitral valve;
   deploying a capture element to seize the leaflet with the capture element;
   with the leaflet seized by the capture element, forming a curved opening in the leaflet proximate the pierced opening with a first cutting element, wherein the curved opening extends around a portion of an axis of the elongated shaft; and
   while seizing the leaflet with the capture element to maintain the relative position of the leaflet relative to the shaft, cutting the seized leaflet with a second cutting element to remove a portion of the leaflet from the mitral valve.
97. A method, comprising:
   advancing a steerable shaft into a cardiovascular system so that its distal end enters a right atrium of a heart and is positioned proximate a superior side of a mitral valve;
   advancing a piercing element into contact with a leaflet of the mitral valve;
   piercing, with the piercing element, the leaflet of the mitral valve to form a pierced opening in the leaflet of the mitral valve; and
   advancing a capture element through the pierced opening in the leaflet to seized the leaflet.
98. A method, comprising:
   advancing a steerable shaft into a cardiovascular system so that its distal end enters a right atrium of a heart and is positioned proximate a superior side of a mitral valve;
      deploying the capture element to seize the leaflet of the valve;
   with the leaflet seized by the capture element, forming a curved cut in the leaflet proximate the pierced opening with a first cutting element, wherein the curved opening extends around a portion of an axis of the elongated shaft; and
   while seizing the leaflet with the capture element to maintain the relative position of the leaflet relative to the shaft, cutting the leaflet with a second cutting element to remove a portion of the leaflet from the mitral valve.
99. A method, comprising:
   advancing a steerable shaft into a cardiovascular system so that its distal end enters an aortic valve and is positioned proximate an inferior side of a leaflet of a mitral valve;
   advancing a piercing element into contact with a leaflet of the mitral valve;
      piercing, with the piercing element, the leaflet of the mitral valve to form a pierced opening in the leaflet of the mitral valve; and
   advancing a capture element through the pierced opening in the leaflet to seized the leaflet.
100. A method, comprising:
   advancing a steerable shaft into a cardiovascular system so that its distal end enters an aortic valve and is positioned proximate an inferior side of a leaflet of a mitral valve;
      deploying the capture element to seize the leaflet of the valve;
   with the leaflet seized by the capture element, forming a curved cut in the leaflet proximate the pierced opening with a first cutting element, wherein the curved opening extends around a portion of an axis of the elongated shaft; and
   while seizing the leaflet with the capture element to maintain the relative position of the leaflet relative to the shaft, cutting the leaflet with a second cutting element to remove a portion of the leaflet from the mitral valve.
101. A method, comprising:
   advancing a steerable shaft into a cardiovascular system so that its distal end is positioned proximate a leaflet of a mitral valve;
   piercing, with a piercing element, the leaflet of the mitral valve to form a pierced opening;
   seizing the leaflet with the capture element;
   with the leaflet seized by the capture element, forming a curved opening in the leaflet;
   while seizing the leaflet with the capture element to maintain the relative position of the leaflet relative to the shaft, cutting, with a cutting element the leaflet; and
      severing mitral chordae tendineae with another cutting element.
102. The method according to any one of paragraphs 93 to 101, using the surgical system according to paragraphs 1 through 47 and 61 through 70.

## Claims

1. A surgical system, comprising:
a shaft having a proximal end and a distal end spaced from the proximal end along a central axis;
a piercing element configured to puncture a leaflet of a valve;
a capture element having a fixed collar and an expandable portion spaced proximal relative to the expandable portion along the central axis, the expandable portion being configured to expand in order to seize a portion of a leaflet of the valve;;
a first cutting element carried by the shaft, the cutting element having a curved cutting edge that extends around a portion of the outer perimeter of the distal end of the shaft, the curved cutting edge configured to create a curved opening in a leaflet of a valve; and
a second cutting element having a first cutter and a second cutter carried at least partly by the shaft, the first cutter and the second cutter being configured to separate outward away from each other while moving in a proximal direction to cut away a portion of the leaflet while the capture element seizes the leaflet of the valve.

2. The surgical system according to claim 1, wherein the curved cutting edge of the cutting element has a C-shape.

3. The surgical system according to claims 1 and 2, wherein the curved cutting edge is an electrode.

4. The surgical system of any one of claim 1, wherein the shaft has an inner channel, wherein the piercing element is movable between a retracted configuration where a distal tip of piercing element is located in the inner channel, and a deployed configuration where the distal tip of the piercing element is located outside of the inner channel.

5. The surgical system of claim 1, wherein a distal tip of piercing element has a circular cross-section with sharp leading edge.

6. The surgical system of claim 1, further comprising at least one actuator configured to move the piercing element along the shaft.

7. The surgical system of claim 1, further comprising an electrode coupled to the piercing element that is responsive to electrical energy.

8. The surgical system of claim 1, further comprising an electrosurgical unit configured to supply electric energy to the electrode.

9. The surgical system of claim 1, further comprising a positioning element carried by the shaft, wherein the positioning element is sized and shaped to stabilize the position of a distal end of the shaft relative to tissue.

10. The surgical system according to claim 1, wherein the shaft has an inner channel, wherein the first cutter and the second cutter of the second cutting element have
a) an insertion configuration, where terminal ends of the first cutter and the second cutter are located inside the inner channel,
b) a deployed configuration, where terminal ends of the first cutter and the second cutter is located outside of the inner channel, and
c) a cutting configuration, where the terminal ends of the first cutter and the second cutter are splayed apart with respect to each other.

11. The surgical system according to claim 10, wherein the cutting element has at least one actuator configured to cause the first cutter and the second cutter to transition through the insertion configuration, the deployed configuration, and the cutting configuration.

12. The surgical system according to any one of the claims 1, 10 and 11, wherein the first cutter and the second cutter each include a) an elongate shaft and b) a cutting hook that extends from the elongate shaft.

13. The surgical system according to claim 1, wherein the capture element includes:
b) an insertion configuration where the first expandable portion and the second expandable portion are collapsed toward the central axis,
c) an expanded configuration where the first expandable portion and the second expandable portion are expanded outwardly away from the central axis, and
d) a captured configuration where at least a portion the first expandable portion and at least a portion of the second expandable portion are positioned to seize a portion of the leaflet while in the expanded configuration.
